(19) Europäisches Patentamt / European Patent Office / Office européen des brevets

(11) **EP 2 165 215 B1**

(12) **EUROPÄISCHE PATENTSCHRIFT**

(45) Veröffentlichungstag und Bekanntmachung des Hinweises auf die Patenterteilung:
**07.05.2014 Patentblatt 2014/19**

(21) Anmeldenummer: **08760032.6**

(22) Anmeldetag: **26.05.2008**

(51) Int Cl.:
***G01T 1/161*** *(2006.01)*

(86) Internationale Anmeldenummer:
**PCT/EP2008/056433**

(87) Internationale Veröffentlichungsnummer:
**WO 2008/142172 (27.11.2008 Gazette 2008/48)**

(54) **BILDERZEUGUNGSAPPARAT UND -METHODE ZUR NUKLEARBILDGEBUNG**

IMAGE FORMATION APPARATUS AND METHOD FOR NUCLEAR IMAGING

APPAREIL DE FORMATION D'IMAGE ET PROCÉDÉ D'IMAGERIE NUCLÉAIRE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **24.05.2007 EP 07010368**
**24.05.2007 EP 07010369**

(43) Veröffentlichungstag der Anmeldung:
**24.03.2010 Patentblatt 2010/12**

(73) Patentinhaber: **SurgicEye GmbH**
**81671 München (DE)**

(72) Erfinder:
• **WENDLER, Thomas**
**81671 München (DE)**
• **NAVAB, Nassir**
**82147 München (DE)**
• **TRAUB, Jörg**
**81371 München (DE)**

(74) Vertreter: **Zimmermann & Partner**
**Postfach 330 920**
**80069 München (DE)**

(56) Entgegenhaltungen:
**EP-A- 1 237 012    US-B1- 6 510 336**

**Beschreibung**

Die vorliegende Erfindung bezieht sich auf Bildeneugungsapparate und Methoden zur Bilderzeugung mit Bilderzeugungsapparaten. Spezielle Ausführungsformen der Erfindung beziehen sich auf Bilderzeugungsapparate zur verbesserten Bilderzeugung durch Qualitätskontrolle, durch Anweisen eines Nutzers zur Datenerhebung und/oder durch fortlaufende Datenerhebung mit verbesserter Verarbeitung. Typische Ausführungsformen der vorliegenden Erfindung beziehen sich auf Bilderzeugungsapparate und -Methoden für medizinische Zwecke.

**HINTERGRUND**

Qualitativ hochwertige Bilderzeugung ist von großem Interesse für einen weiten Bereich von Anwendungen. Insbesondere im medizinischen Bereich, wo die Gesundheit eines Patienten davon abhängen kann, ist eine bestmögliche Bilderzeugung beispielsweise als Basis für Operationen am Patienten erforderlich.

Für gewöhnlich werden medizinische Bilder entweder präoperativ oder intraoperativ erzeugt. Auch ein Registrieren von Bildern ist bekannt, beispielsweise das Registrieren eines anatomischen Bildes mit einem funktionellen Bild, d.h., einem Bild, dass Körperaktivität sichtbar macht. Solche registrierten Bilder können beispielsweise bei Tumoroperationen helfen zu entscheiden, welche Gewebeteile herauszuschneiden sind. Wünschenswert sind möglichst aktuelle und hochwertige Bilder, da so vermieden werden kann, gesundes Gewebe zu schädigen oder krankes nicht zu entfernten.

US 6,510,336 beschreibt eine Identifizierung von kranken Zellen während einer Operation, wobei eine Nuktearsonde verwendet wird. Eine Diagnoseelektronik kann einen optischen Bilderzeuger zum Erstellen eines optischen und eines Strahlungsbildes umfassen. US 6,510,336 beschreibt die Berechnung einer z-Punktezahl während eines laufenden 5-Sekundenfensters als einer Zahl von Standardabweichungen über oder unter dem Mittel der Zählrate. Wird eine z-Punktezahl von größer als 3 berechnet, kann ein Audiosignal ausgegeben werden, das den Operierenden auf Strahlungssicnalspitzen aufmerksam macht, die ihm sonst entgehen könnten.

EP 1 237 012 beschreibt eine Gammakamera mit einem Flächensensor mit 16x16 Halbleiterdetektionselementen und Anzeigeelementen auf der Rückseite der Kamera, um dem Bediener der Kamera anzuzeigen, in welcher Richtung sich die Strahlungsquelle befindet. So kann der Bediener zuverlässig die Strahlungsquelle ins Zentrum der Kamera bringen.

Hochwertige Bilder zu erzeugen stellt hohe Anforderungen an Detektordaten zur Bilderzeugung und an ein Auswertesystem, das diese Daten verarbeiten muss. Das gilt besonders für die Verarbeitung von Detektordaten mit beweglichen Detektoren, die beispielsweise in der Hand getragen werden.

Folglich besteht Bedarf an der Verbesserung der Erhebung und Auswertung von Detektordaten und an verbesserter Bilderzeugung.

**ZUSAMMENFASSUNG**

Im Lichte der obigen Ausführungen wird ein Verfahren zur Bilderzeugung gemäß den Ansprüchen 1 bis 9 und ein Bilderzeugungsapparat gemäß den Ansprüchen 10 bis 15 bereitgestellt.

Gemäß einem Aspekt der Erfindung wird ein Bilderzeugungsapparat zur Bilderzeugung bereitgestellt. Der Bilderzeugungsapparat umfasst einen beweglichen Detektor zur Detektion von radioaktiver Strahlung während eines Detektionszeitraums. Der Bilderzeugungsapparat umfasst weiter ein Auswertesystem. Das Auswertesystem umfasst ein Schnittstellensystem zur Überrnittlung von Detektordaten an das Auswertesystem. Die Detektordaten umfassen Informationen über die detektierte radioaktive Strahlung und über die Position und/oder Orientierung des Detektors zur Bilderzeugung. Das Auswertesystem umfasst weiter einen Datenspeicherabschnitt zum Speichern der Detektordaten. Das Auswertesystem umfasst weiter einen Programmspeicherabschnitt mit einem Programm zum wiederholten Ermitteln mindestens eines Qualitätswerts hinsichtlich der Bilderzeugung aus den Detektordaten während des Detektionszeitraums. Der Bilderzeugungsapparat umfasst ein Ausgabesystem, welches mindestens eine Ausgabeeinheit umfasst. Die mindestens eine Ausgabeeinheit umfasst eine Ausgabeeinheit zum Ausgeben einer Anweisung an einen Nutzer zum weiteren Bewegen des Detektors in Abhängigkeit von dem mindestens einen ermittelten Qualitätswert. Die Anweisung beziehen sich zumindest auf einen Teil des verbleibenden Detektionszeitraums.

Einem weiteren Aspekt der Erfindung entsprechend wird ein Verfahren zur Bilderzeugung durch einen Bilderzeugungsapparat bereitgestellt. Das Verfahren umfasst das Detektieren von radioaktiver Strahlung durch einen beweglichen Detektor während eines Detektionszeitraums. Das Verfahren umfasst weiter das Sammeln von Detektordaten zur Bilderzeugung durch ein Auswertesystem des Bilderzeugungsapparats. Die Detektordaten umfassen Informationen über die detektierte Strahlung und über die Position und/oder Orientierung des Detektors. Das Verfahren umfasst weiter ein wiederholtes Ermitteln mindestens eines Qualitätswerts aus den gesammelten Detektordaten durch das Auswertesystem während des Detektionszeitraums und Ausgeben einer Anweisung an einen Nutzer zum weiteren Bewegen des Detektors in Abhängigkeit von dem mindestens einen ermittelten Qualitätswert, wobei sich die Anweisung zumindest auf einen Teil des verbleibenden Detektionszeitraums bezieht.

Weitere Merkmale, Aspekte und Details, die mit hier beschriebenen Ausführungsformen kombiniert werden können, werden in den abhängigen Ansprüchen, der Beschreibung und den Abbildungen offenbart.

## KURZBESCHREIBUNG DER ABBILDUNGEN

Damit die zuvor aufgeführten Merkmale im Detail besser verstanden werden können, wird eine speziellere Beschreibung mit Bezug auf Ausführmngsformen der Erfindung gegeben. Die beigefügten Abbildungen beziehen sich auf Ausführungsformen der Erfindung und werden im folgenden kurz beschrieben:

Abbildung 1 zeigt einen schematischen Aufbau eines Bilderzeugungsapparats gemäß Ausführungsformen der Erfindung;

Abbildung 2 zeigt ein Detektorsystem des Bilderzeugungsapparats gemäß Ausführungsformen der Erfindung;

Abbildung 3 zeigt ein Erfassungssystem des Bildeizeugungsapparats gemäß Ausführungsformen der Erfindung;

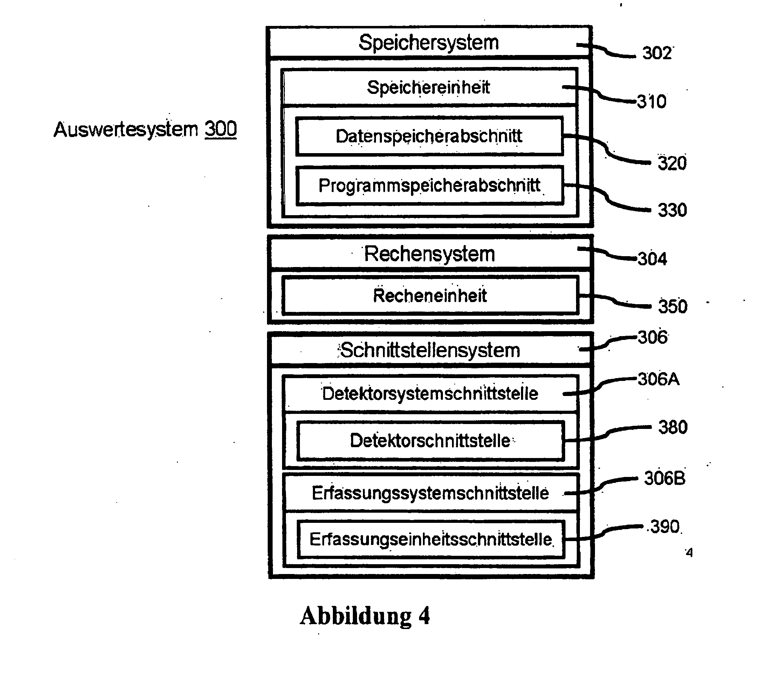

Abbildung 4 zeigt den schematischen Aufbau eines Auswertesystems des Bilderzeugungsapparats gemäß Ausführungsformen der Erfindung;

Abbildung 5 zeigt den schematischen Aufbau von Programmspeicherabschnitten des Auswertesystems gemäß Ausführungsformen der Erfindung;

Abbildung 6 zeigt ein Ausgabesystem des Bilderzeugungsapparats gemäß Ausführungsformen der Erfindung;

Abbildung 7 zeigt ein weiteres Ausgabesystem des Bilderzeugungsapparats gemäß Ausführungsformen der Erfindung;

Abbildung 8 zeigt ein Führungssystem des Bilderzeugungsapparats gemäß Ausführungsformen der Erfindung;

Abbildung 9 zeigt einen Bilderzeugungsapparat gemäß Ausführungsformen der Erfindung beim Einsatz im medizinischen Bereich;

Abbildung 10 zeigt die Erstellung eines Detektionsmodells gemäß Ausführungsformen der Erfindung;

Abbildung 11 zeigt die Erstellung eines Detektionsmodells über Messungen gemäß Ausführungsformen der Erfindung;

Abbildung 12 zeigt einen Qualitätskontrollprozess gemäß Ausführungsformen der Erfindung;

Abbildung 13 zeigt einen iteratives Flussdiagramm mit einem Schritt des Anweisens eines Nutzers gemäß Ausführungsformen der Erfindung;

Abbildung 14 zeigt einen Detektionsprozess mit einem frei beweglichen Detektor gemäß Ausführungsformen der Erfindung.

## AUSFÜHRLICHE BESCHREIBUNG

Im Folgenden wird detailliert Bezug genommen auf verschiedene Ausführungsformen der Erfindung, von denen einige beispielhaft durch die Abbildungen illustriert werden. Jedes Beispiel wird zur Erklärung und zum besseren Verständnis der Erfindung gegeben und soll nicht als Beschränkung der Erfindung aufgefasst werden. So können Merkmale, die in einer Ausführungsform beschrieben oder illustriert werden, in Verbindung mit anderen Ausführungsformen gebracht werden, um neue Ausführungsformen zu erzeugen. Beabsichtigt ist, dass solche Modifikationen und Variationen umfasst sind.
Insbesondere werden Ausführungsformen der Erfindung zum besseren Verständnis meist mit Bezug auf eine Bildeizeugung für medizinische Zwecke beschrieben. Jedoch können viele der Ausführungsformen auch für die Bilderzeugung in anderen Bereichen verwendet werden.
Innerhalb der folgenden Beschreibung und in den Abbildungen beziehen sich gleiche Referenzzeichen auf gleiche oder ähnliche Komponenten. Im Allgemeinen werden nur Unterschiede bezüglich individueller Ausführrungsformen explizit beschrieben.
Der Ausdruck "Detektionszeitraum", der hier verwendet wird, bezeichnet einen Zeitraum zwischen dem Beginn eines ersten Detektionsprozesses und dem Ende eines letzten Detektionsprozesses. Der erste und letzte Detektionsprozess können identisch sein, so dass der Detektionszeitraum ein Zeitraum ist, während welchem andauernd ein Detektionsprozess stattfindet. Die erste und letzte Detektion können auch verschieden sein. In einen Detektionszeitraum können daher andere Prozesse fallen. Beispielsweise können solche anderen Prozesse Datenauswerteprozesse sein. Der mindestens eine Detektionsprozess, der in dem Detektionszeitraum stattfindet, wird von demselben Detektor bzw. Detektorsystem an demselben Objekt ausgeführt. Ein Beispiel für einen Detektionszeitraum ist der Zeitraum zwischen dem ersten Messen von radioaktiver Strahlung mit einer Gamma-Sonde an einem Patienten und dem letzten Messen, wobei beispielsweise nach der letzten Messung ein letztes Bild mit der Darstellung von Körperfunktionen erzeugt werden kann. Zwischen dem ersten Messen und dem letzten Messen können durchaus auch eine oder mehrere Messpausen liegen, beispielsweise zur Datenauswertung oder gar zum Messen an einem anderen Objekt. Ein Detektionszeitraum wäre beispielsweise nicht definiert durch ein erstes Messen nur am Bein eines Patienten und durch ein weiteres Messen nur am Bauch des Patienten.
Die Angabe, dass eine Handlung "während eines Detektionszeitraums" ausgeführt wird (oder allgemeiner während eines beliebigen Zeitraums), ist ferner nicht so zu verstehen, dass sie den gesamten Zeitraum ausfüllen müsste. Sie kann vielmehr auch nur während eines Teils des Detektionszeitraums stattfinden. Die Handlung kann auch unterbrochen sein.

Der Ausdruck "frei beweglich" wird hier im Allgemeinen so verstanden, dass die Position und/oder Orientierung eines Objekts, das frei beweglich ist, im Wesentlichen beliebig verändert werden kann. Beispielsweise ist ein Detektor, der in der Hand tragbar ist, ein frei beweglicher Detektor. Auch ein Detektor, der an einem Roboterarm mit genügend vielen Freiheitsgraden befestigt ist, ist frei beweglich, wobei der Roboterarm beispielsweise von einem Nutzer kontrolliert wird. Ein Detektor, der nur entlang einer Schiene fahrbar ist, ist beweglich, aber nicht frei beweglich.

Der Ausdruck "fortlaufend" umfasst in Bezug auf eine Handlung, z.B. "fortlaufendes Sammeln von Detektordaten", eine andauernde oder regelmäßig wiederholte Handlung. Die zeitlichen Abstände zwischen den regelmäßige Wiederholungen können im Prinzip beliebig kurz sein, also quasi-kontinuierlich. Jedoch ist für den Fachmann offensichtlich, dass z.B. physikalische Zwänge einem beliebig kurzen Abstand entgegenstehen können. Beispielsweise können Detektoren sogenannte "Totzeiten" aufweisen, so dass während solcher Totzeiten keine Detektion stattfinden kann. Folglich wäre beispielsweise auch beim fortlaufenden Sammeln von Detektordaten eine regelmäßige Wiederholung einer Datenaufnahme innerhalb des Sammelvorgangs nicht innerhalb von Zeitabständen möglich, die kleiner sind als die genannten Totzeiten. Der Begriff "fortlaufend" umfasst in Bezug auf eine Handlung aber auch eine Wiederholung oder mehrmalige Wiederholung in willkürlichen kurzen Zeitabständen. Auch willkürliche Zeitabstände können im Prinzip beliebig kurz aufeinanderfolgend sein und Einschränkungen wie die oben ausgeführten gelten analog.

Das "Erzeugen eines Bildes" umfasst die Erzeugung von Bilddaten, ohne dass es notwendigerweise der Ausgabe dieser Bilddaten auf einer Ausgabeeinheit, z.B. auf einem Monitor, bedarf.

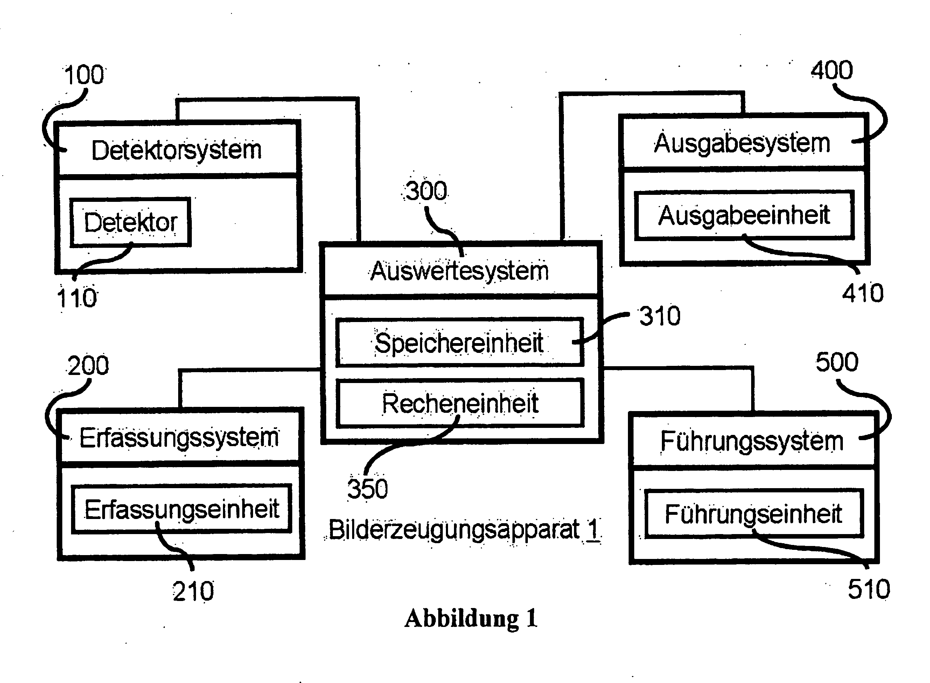

Abb. 1 zeigt einen Bilderzeugungsapparat 1 gemäß Ausführungsformen der Erfindung. Wie in Abb. 1 zu sehen, umfasst der Bilderzeugungsapparat 1 ein Detektorsystem 100. Das Detektorsystem 100 umfasst mindestens einen Detektor 110. Der Bilderzeugungsapparat umfasst weiter ein Auswertesystem 300. Das Auswertesystem 300 umfasst mindestens eine Speichereinheit 310 und mindestens eine Recheneinheit 350. In einigen Ausführungsformen sind das Detektorsystem und das Auswertesystem durch ein Datenaustauschsystem 20 verbunden. Gemäß weiteren Ausführungsformen umfasst der Bilderzeugungsapparat ein Erfassungssystem 200 wie in Abb. 1 gezeigt. Das Erfassungssystem 200 umfasst mindestens eine Erfassungseinheit 210. In weiteren Ausführungsformen umfasst der Bilderzeugungsapparat ein Ausgabesystem 400. Das Ausgabesystem umfasst mindestens eine Ausgabeeinheit 410. In einigen Ausführungsformen sind das Erfassungssystem 200 und das Ausgabesystem 400 mit dem Auswertesystem 300 durch ein Datenaustauschsystem verbunden. In weiteren Ausführungsformen umfasst der Bilderzeugungsapparat ein Führungssystem 500. Das Führungssystem 500 umfasst mindestens eine Führungseinheit 510. Das Führungssystem kann mit dem Auswertesystem durch das Datenaustauschsystem verbunden sein. Die einzelnen Systeme werden im Folgenden näher beschrieben.

Detektorsystems 100

Erfindungsgemäß umfasst das Detektorsystem 100 einen Detektor 110. Erfindungsgemäß ist der Detektor 110 ein Strahlungsdetektor, nämlich ein Detektor für radioaktive Strahlung. Erfindungsgemäß ist der Detektor beweglich, sogar frei beweglich. In typischen Ausführungsformen ist der Detektor in der Hand tragbar. Der Detektor kann eine Gammastrahlensonde, eine Betastrahlensonde, eine Compton-Sonde, eine Gammastrahlenkamera, eine Gammastrahlenminikamera, eine Betastrahlenkamera oder eine Compton-Kamera sein. Der Detektor kann auch ein Detektor

von Röntgenstrahlung oder ein Detektor anderer radioaktiver Strahlung sein.

Detektordaten können Informationen über die detektierte Strahlung umfassen. Die Detektordaten können in gewissem Umfang aufbereitet sein, im Allgemeinen sollte aber noch eine Zuordnung einzelner Datensätze zu bestimmten Detektionsereignissen oder zumindest zu einer Gruppe von Detektionsereignissen möglich sein. Die Detektordaten umfassen auch Position und/oder Orientierung des Detektors. Detektordaten können darüberhinaus weitere Daten umfassen.

Das Detektorsystem 100 kann beispielhaft mindestens einen weiteren Detektor umfassen. Der mindestens eine weitere Detektor kann dem Detektor 110 gleich oder ähnlich sein. Der mindestens eine weitere Detektor kann auch von dem Detektor 110 verschieden sein. Der mindestens eine weitere Detektor kann beispielsweise auch eine Ultraschallsonde, ein Röntgendetektor, eine optische Kamera, ein optisches Mikroskop, eine Fluoreszenzkamera, eine Autofluoreszenzkamera, ein Magnetresonanztomografiedetektor, ein Positronemissionstomografiedetektor, kurz PET, ein Einzelphotonemissionscomputertomografiedetektor, kurz SPECT oder ein anderer Detektor sein.

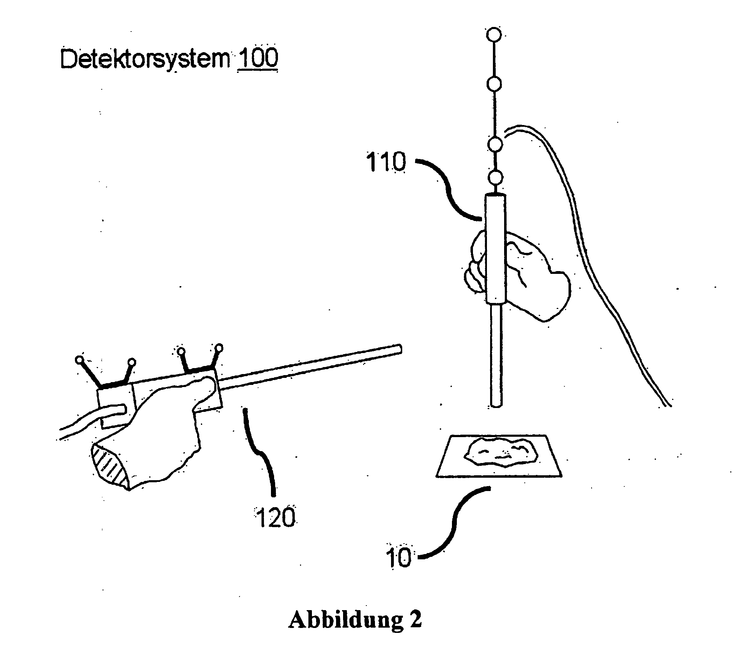

Abb. 2 zeigt ein Detektorsystem 100 gemäß Ausführungsformen der vorliegenden Erfindung. In Abb. 2 sind zwei Detektoren 110, 120 des Detektorsystems 100 gezeigt: eine Sonde 110 zum Detektieren radioaktiver Strahlung und eine optische Kamera 120. Die radioaktive Strahlung kann beispielsweise gamma-, beta-, Compton-, Röntgen- oder Alphastrahlung sein. Weiter ist eine zu detektierende radioaktive Strahlenquelle 10 abgebildet. Eine Strahlenquelle kann im Allgemeinen hier und im Folgenden eine räumlich verteilte Strahlenquelle sein, also eine räumliche Strahlungsverteilung. Eine Strahlenquelle kann aber auch eine im Wesentlichen zweidimensionale Strahlenquelle sein, d.h. eine im Wesentlichen flächige Strahlungsverteilung.

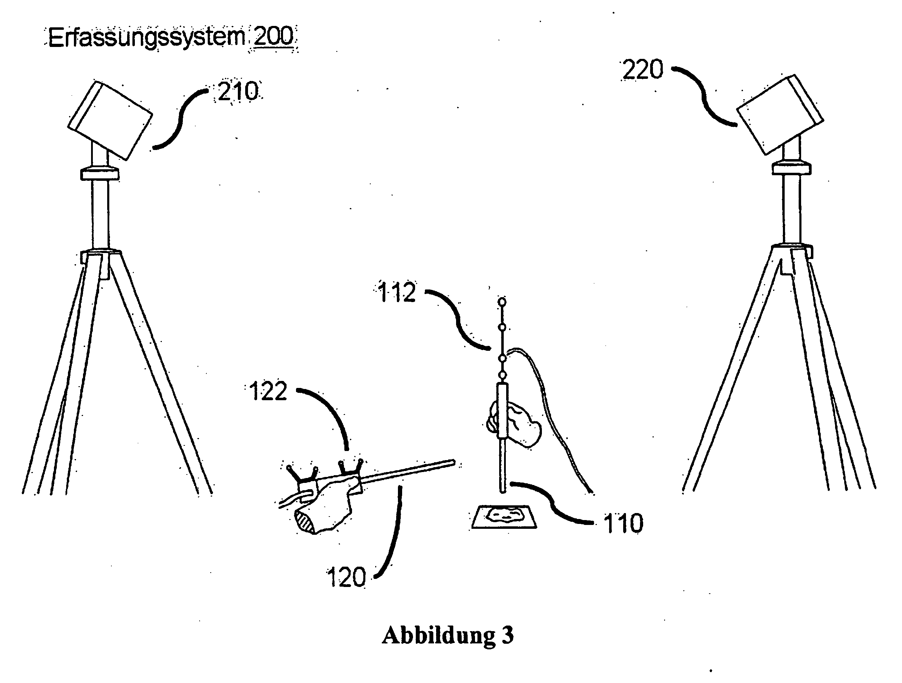

Die Detektoren können wie dargestellt in der Hand gehalten werden und sind in den drei Raumrichtungen beweglich und orientierbar, d.h. frei beweglich. Weiter weisen die Detektoren 110, 120 jeweils ein Kabel zur Energieversorgung und zum Datenaustausch etwa mit dem in Abb. 1 dargestellten Auswertesystem 300 auf. Weiter weisen die Detektoren 110, 120 jeweils Markierungen zur Erfassung durch das in Abb. 3 dargestellte Erfassungssystem 200 auf, wie weiter unten zu Abb. 3 beschrieben. Es kann auch ein Erfassungssystem 200 vorgesehen werden, das ohne Markierungen auskommt, wie weiter unten beschrieben.

Detektordaten wie Detektordaten mit Information über gemessene Strahlung und über die Position und/oder Orientierung der Detektors können dem Auswertesystem 300 (s. Fig. 1) zur Verfügung gestellt werden. Insbesondere kann das Auswertesystem 300 die Detektordaten sammeln.

### Erfassungssystem 200

Einigen Ausführungsformen entsprechend umfasst der Bilderzeugungsapparat ein Erfassungssystem 200. Gemäß einigen Ausführungsformen umfasst das Erfassungssystem 200 eine Erfassungseinheit 210. Die Erfassungseinheit kann eine optische, elektromagnetische, mechanische, Roboter gestützte, Radiowellen gestützte, Schallwellen gestützte, Goniometer gestützte, Potenziometer gestützte, Gyroskop gestützte, Beschleunigungsmesser gestützte, strahlungsgestützte, Röntgenstrahlung gestützte oder eine Infrarot- oder Weißlichterfassungseinheit sein oder eine andere Erfassungseinheit. Gemäß weiteren Ausführungsformen umfasst das Erfassungssystem 200 eine weitere Erfassungseinheit 220 oder weitere Erfassungseinheiten. Die Erfassungseinheit 220 oder die weiteren Erfassungseinheiten können Erfassungseinheiten wie die oben aufgezählten oder andersartige Erfassungseinheiten sein. Um eine Machbarkeit oder Zuverlässigkeit des Erfassungssystems zu gewährleisten, enthalten Ausführungsformen mindestens zwei, mindestens drei oder mindestens vier Erfassungseinheiten.

Abb. 3 zeigt ein Erfassungssystem 200 gemäß typischen Ausführungsformen der vorliegenden Erfindung. Abb. 3 zeigt zwei optische Erfassungseinheiten 210 und 220. Die optischen Erfassungseinheiten 210 und 220 erfassen Markierungen 112 an der Sonde radioaktiver Strahlung 110 und Markierungen 122 an der optischen Kamera 120. Die optischen Erfassungseinheiten 210 und 220 erzeugen durch das Erfassen der Markierungen 112 und 122 Daten mit Information über die Position und/oder Orientierung der Sonde 110 und der Kamera 120. In dem in Abb. 3 dargestellten Beispiel sind die optischen Erfassungseinheiten 210 und 220 genau justiert, und die Position und Orientierung der Sonde 110 und der Kamera 120 wird durch Erfassen der Position der Markierungen 112 bzw. 122 mittels bekannter Triangulationsverfahren ermittelt.

Daten des Erfassungssystems wie Detektordaten mit Information über Position und/oder Orientierung können dem Auswertesystem 300 zur Verfügung gestellt werden. Insbesondere kann das Auswertesystem 300 solche und andere Detektordaten sammeln.

### Auswertesystem 300

Beispielhaft umfasst das Auswertesystem 300 ein Speichersystem 302 mit einer Speichereinheit 310. Die Speichereinheit 310 kann beispielsweise eine Computerfestplatte oder ein anderes Massenspeichergerät sein oder von anderer Art sein. Gemäß einem Aspekt der Erfindung umfasst die Speichereinheit 310 einen Datenspeicherabschnitt 320. Der Datenspeicherabschnitt 320 wird beispielweise zum Speichern von Detektordaten verwendet. Der Datenspeicherabschnitt 320 kann auch zum Speichern anderer Daten verwendet werden. Gemäß einem Aspekt der Erfindung umfasst die Speichereinheit 310 einen Programmspeicherabschnitt 330. Der Programmspeicherabschnitt 330 sowie, gemäß weiteren Ausführungsformen, weitere Programmspeicherabschnitte werden weiter unten beschrieben. Die Datenspeichereinheit 310 kann weitere Datenspeicherabschnitte und weitere Programmspeicherabschnitte umfassen. Die verschiedenen Speicherabschnitte müssen nicht physikalische oder speichertechnische Einheiten bilden; verschiedene Abschnitte werden vielmehr lediglich in Bezug auf die Natur der darin gespeicherten oder zu speichernden Daten unterschieden. Das Speichersystem 302 kann weitere Speichereinheiten umfassen. Die weiteren Speichereinheiten können der Speichereinheit 310 ähnlich sein oder von anderer Art sein.

Beispielhaft umfasst das Auswertesystem 300 ein Rechensystem 304. Das Rechensystem 304 umfasst gemäß einigen Ausführungsformen eine Recheneinheit 350. Die Recheneinheit 350 kann beispielsweise der rechnende Teil eines Computers sein, z.B. ein Prozessor. Gemäß weiteren Ausführungsformen umfasst das Rechensystem 304 weitere Recheneinheiten, die der Recheneinheit 350 ähnlich sein oder von anderer Art sein können. Insbesondere können mindestens eine Recheneinheit und mindestens eine Speichereinheit in speziellen Geräten wie beispielsweise handelsüblichen Computern integriert sein.

Gemäß einem weiteren Aspekt der Erfindung umfasst das Auswertesystem ein Schnittstellensystem 306. In einigen Ausführungsformen umfasst das Schnittstellensystem 306 eine Detektorsystemschnittstelle 306A mit einer Detektorschnittstelle 380 zum Datenaustausch mit einem Detektor, beispielsweise mit dem Detektor 110. In weiteren Ausführungsformen umfasst das Schnittstellensystem 306 ein Erfassungssystemschnittstelle 306B mit einer Erfassungseinheitenschnittstelle 390 zum Datenaustausch mit einem Erfassungssystem (z.B: dem Erfassungssystem 200 aus Abb. 3). Ein Schnittstellensystem 306 oder Teile davon können ebenfalls in speziellen Geräten wie einem handelsüblichen Computer integriert sein. In einigen Ausführungsformen kommuniziert das Auswertesystem mit anderen Teilsystemen des Bilderzeugungsapparats über solche Schnittstellensysteme mit Hilfe eines Datenaustauschsystems.

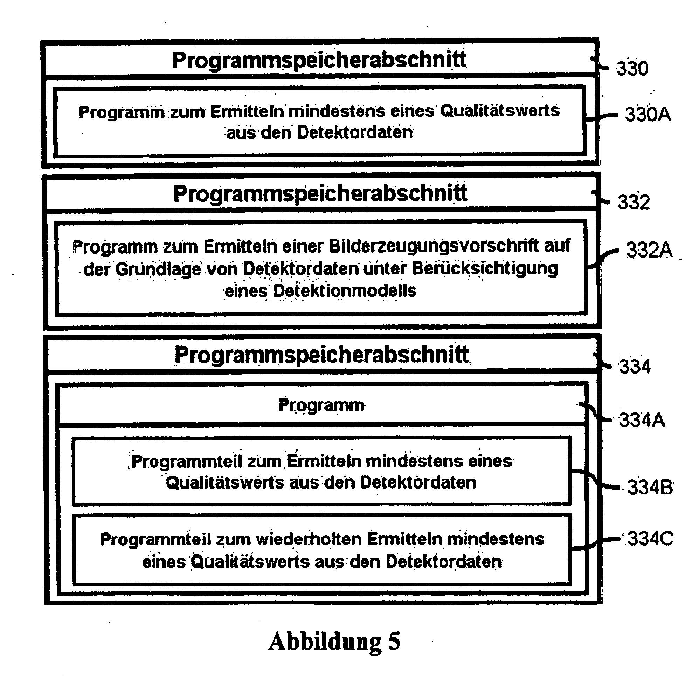

In weiteren Ausführungsformen der Erfindung umfasst der Programmspeicherabschnitt 330 ein Programm. Wie in Abbildung 5 gezeigt kann das Programm beispielsweise ein Programm 330A zum Ermitteln mindestens eines Qualitätswerts auf der Grundlage von Detektordaten sein. In anderen Ausführungsformen umfasst eine Speichereinheit weitere Programmspeicherabschnitte, beispielsweise weitere Programmspeicherabschnitte 332 und 334 mit Programm 332A zum Ermitteln einer Bilderzeugungsvorschrift auf der Grundlage von Detektordaten unter Berücksichtigung eines Detektionsmodells, beziehungsweise mit Programm 334A. Programm 334A umfasst Programmteil 334B zum Ermitteln mindestens eines Qualitätswerts auf der Grundlage von Detektordaten und Programmteil 334C zum wiederholten Ermitteln mindestens eines Qualitätswerts auf der Grundlage von Detektordaten.

Insbesondere können Programme, die z.B. ähnliche Funktionen ausführen, auch als Programmteile eines einzigen Programms gestaltet werden wie beispielsweise oben für Programm 334A beschrieben. Das Gleiche gilt auch für funktionell verschiedene Programme. In beiden Fällen gilt, dass für einen ersten Programmabschnitt mit einem ersten Programm und einen zweiten Programmabschnitt mit einem zweiten Programm, die bereitgestellt werden, der erste und zweite Programmabschnitt identisch sind, und das erste und zweite Programm als Teile eines einzigen Programms aufgefasst werden.

Weiter können in Ausführungsformen, in denen ein erster Programmabschnitt mit einem ersten Programm und ein zweiter Progammabschnitt mit einem zweiten Programm bereitgestellt werden, sowohl der erste Programmabschnitt mit dem zweiten identisch sein als auch das erste Programm mit dem zweiten.

## Ausgabesystem 400

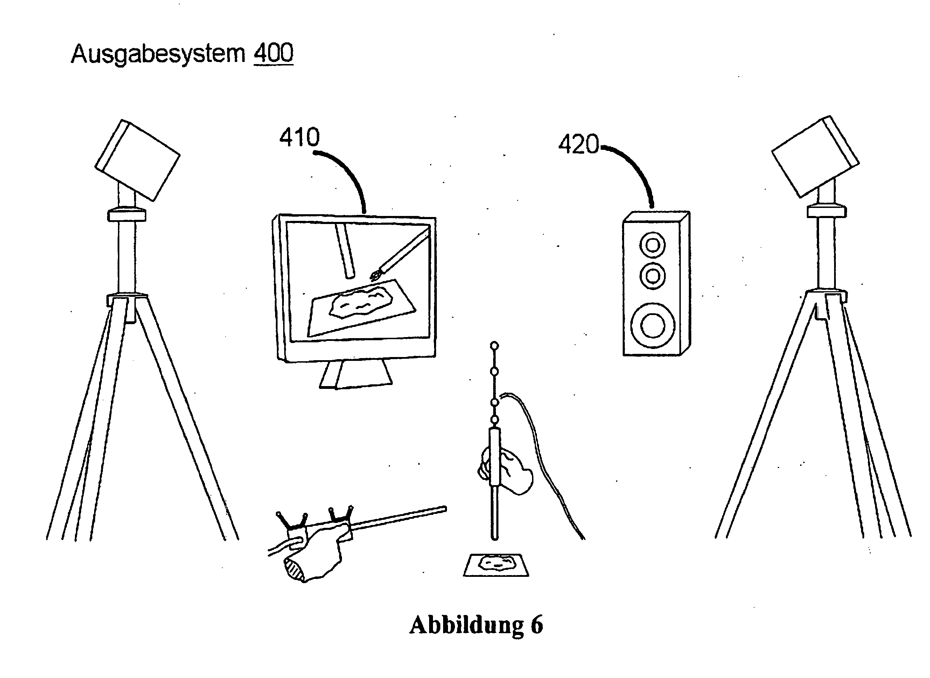

Mit Bezug auf Abb. 6 umfasst der Bilderzeugungsapparat gemäß einem weiteren Aspekt der Erfindung ein Ausgabesystem 400. Das Ausgabesystem 400 umfasst eine Ausgabeeinheit 410. Die Ausgabeeinheit 410 kann eine visuelle, akustische oder haptische Ausgabeeinheit sein oder eine Kombinationsform davon. In einem Aspekt der Erfindung ist die Ausgabeeinheit 410 eine Ausgabeeinheit zum Anzeigen von Bildern oder einer Anweisung an einen Nutzer. Ein Nutzer ist in der Regel ein Mensch. Alternativ kann ein Nutzer aber auch ein anderes Lebewesen oder ein unbelebtes Objekt sein, z.B. eine Maschine.

In weiteren Ausführungsformen umfasst das Ausgabesystem 400 weitere Ausgabeeinheiten. Diese können von ähnlicher Art sein wie die Ausgabeeinheit 410 oder von anderer Art.

Ausgabeeinheiten können realitätsabbildend sein, eine virtuelle Realität abbilden oder eine erweiterte Realität abbilden. Eine Ausgabeeinheit einer erweiterten Realität kann beispielsweise ein reales Bild mit virtuellen Bildern kombinieren.

Beispielhaft kann eine Ausgabeeinheit unter anderen eine der folgenden sein: Monitor, optisch durchscheinender Monitor, Stereomonitor, am Kopf befestigte Stereodisplays, akustische frequenzcodierte Rückkopplungssysteme, akustische pulscodierte Rückkopplungssysteme, Kraftrückkopplungsjoysticks oder Kraftdrehmomentrückkopplungsjoysticks oder andere Arten von visuellen, akustischen und/oder haptischen Ausgabeeinheiten oder Kombinationen davon.

Abb. 6 zeigt eine Ausgabeeinheit 410. In Abb. 6 ist die Ausgabeeinheit 410 eine optische Ausgabeeinheit, speziell ein Monitor. Abb. 6 zeigt weiter eine akustische Ausgabeeinheit 420. In Abb. 6 ist die akustische Ausgabeeinheit ein Lautsprecher.

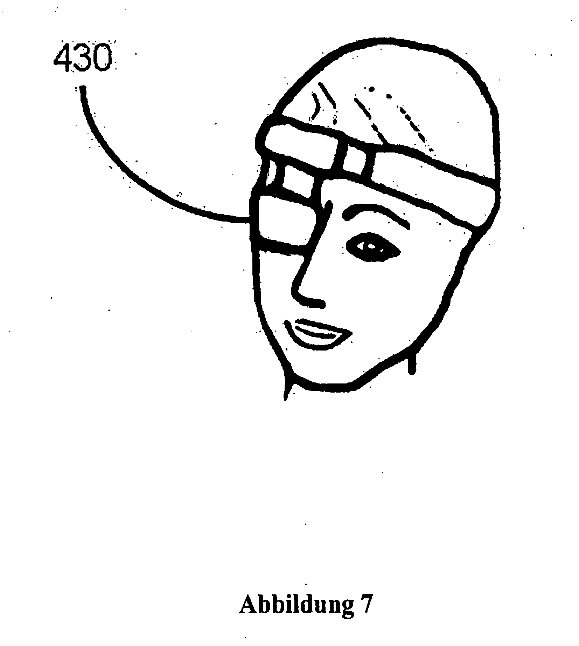

Abb. 7 zeigt eine Abbildungseinheit 430 in Form eines kopfmontierten visuellen Displays.

## Führungssystem 500

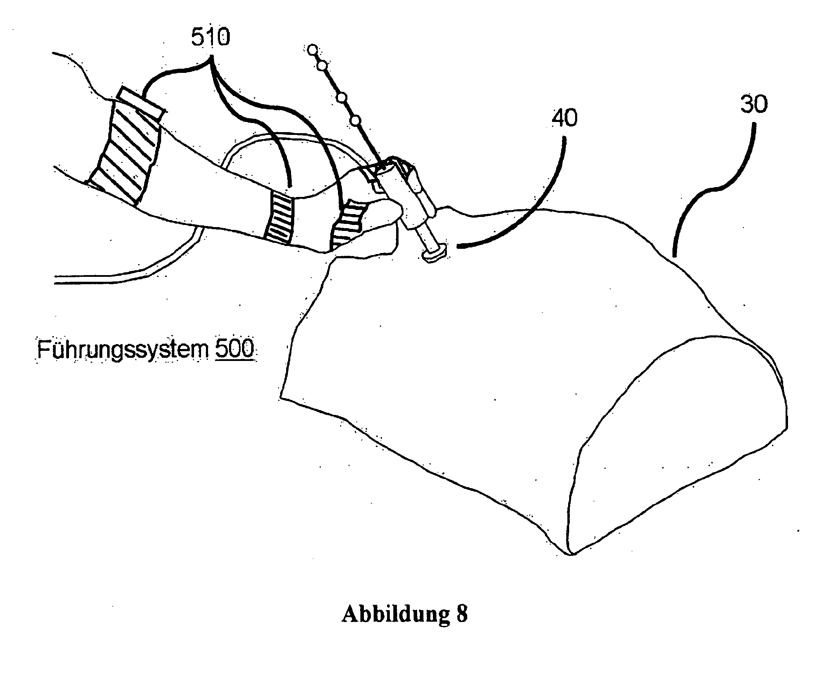

Beispielhaft umfasst der Bilderzeugungsapparat ein Führungssystem 500, wie etwa in Abb. 8 dargestellt. Das Führungssystem 500 umfasst eine Führungseinheit 510. Eine Führungseinheit 510 kann beispielsweise ein Objekt durch einen Roboterarm führen. Die Führungseinheit 510 kann auch einen Nutzer führen. Das Führen kann ebenfalls robotergestützt sein oder aber auf optischen, akustischen oder haptischen Signalen oder auf einer Kombination davon beruhen. Die in Abb. 8 dargestellte Führungseinheit 510 führt einen Nutzer durch haptische Signale. In Abb. 8 dient die Führungseinheit 510 dem besseren Führen eines chirurgischen Instruments 40 beim Eingriff in einen Körper 30. Die Führungseinheit gibt beispielsweise einen Widerstand, sei es durch mechanische Hinderung oder durch Reizung von Muskeln mittels elektrischer Impulse.

Die Führungseinheit 510 oder weitere Führungseinheiten können auch durch eine Ausgabeeinheit des Ausgabesystems gebildet werden, wenn die Führung des Benutzers durch eine entsprechende Ausgabe bewerkstelligt wird. Das Führungssystem 500 kann daher sogar identisch mit dem Ausgabesystem 400 sein.

Beispielhaft umfasst der Bilderzeugungsapparat ein Datenaustauschsystem. Wie in Abb. 1 zu sehen, dient das Datenaustauschsystem dem Austausch von Daten zwischen Systemen des Bilderzeugungsapparats, beispielsweise dem Austausch von Daten zwischen Detektorsystem und Auswertesystem, zwischen Erfassungssystem und Auswertesystem, zwischen Ausgabesystem und Auswertesystem, oder zwischen Führungssystem und Auswertesystem (wie in Abb. 1 durch entsprechende Verbindungslinien dargestellt ist). Das Datenaustauschsystem kann sich in einigen Ausführungsformen auf Schnittstellen wie die Detektorschnittstelle 380 oder die Erfassunassystemschnittstelle 390 stützen. Allgemein kann der Austausch von Daten durch eine Verbindung der Systeme erfolgen mit Hilfe von Leitungen oder aber kabellos oder auf sonst eine Weise.

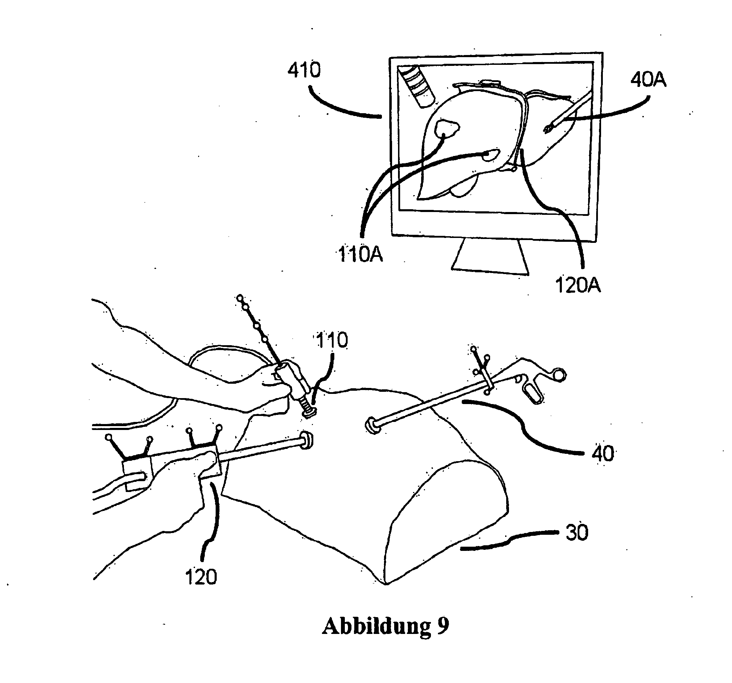

Abbildung 9 zeigt einen Körperteil eines Menschen oder anderen Lebewesens, in welchen radioaktive Substanzen eingebracht worden sind, sogenannte Tracer, die sich in bestimmten Regionen bevorzugt anreichern oder die dort stecken bleiben. Die Regionen oder räumlichen Gebiete, in denen die radioaktiven Substanzen angereichert bzw. steckengebliebensind, können als abgeschlossene Gebiete aufgefasst werden, die eine Quelle von radioaktiver Strahlung umfassen.

Abb. 9 zeigt weiter einen Detektor für radioaktive Strahlung 110. Der Detektor 110 misst die radioaktive Strahlung, die von der Quelle innerhalb des Körpers ausgeht. Weiter zeigt Abb. 9 ein Laparoskop 120, das Daten zur Erzeugung eines optischen Bildes des Körperinneren aufnimmt. Die von dem Detektor für radioaktive Strahlung 110 und dem Laparoskop 120 aufgenommenen Daten werden in dem Auswertesystem (nicht gezeigt) gesammelt und verarbeitet. Weiter werden die Positionen und/oder Orientiemngen der beiden Detektoren über Markierungen 112 und 122 erfasst, und die entsprechenden Daten werden vom Auswertesystem gesammelt. Aus allen diesen Daten erzeugt das Auswertesystem anhand einer Bilderzeugungsvorschrift sowohl ein optisches Bild des Körperinneren aufgrund der Daten des Laparoskops als auch ein funktionelles Bild, das Körperfunktionen wie Stoffwechsel sichtbar macht, aufgrund der Daten des Detektors für radioaktive Strahlung. Das Bild kann insbesondere dreidimensional sein.

Auf einer Ausgabeeinheit 410 werden das optische, anatomische Bild und das funktionelle Bild überlagert und z.B. dreidimensional dargestellt. Die Überlagerung wurde aufgrund einer Registrierung des optischen Bildes mit dem anatomischen Bild durch das Auswertesystem erzeugt.

Weiter zeigt Abb. 9 ein chirurgisches Instrument 40, dessen Position und/oder Orientierung ebenfalls erfasst werden. Die erfassten Daten des chirurgischen Instruments werden ebenfalls durch das Auswertesystem verarbeitet. Auf diese Weise kann ein Bild des chirurgischen Instruments und seiner Lage im Körperinneren durch die Auswerteeinheit bestimmt werden. Dieses Bild kann mit dem anatomischen und optischen Bild ebenfalls registriert werden und auf der Auswerteeinheit 410 angezeigt werden.

Ist insbesondere das funktionelle Bild hochwertig und aktuell, und ist die Registrierung mit dem optischen Bild und dem Instrumentenbild gut, ermöglicht die Ausgabe der registrierten Bilder auf der Anzeigeeinheit einem Operierenden eine präzise Kontrolle des Eingriffs.

Jedoch sind die Bilder vorbekannter Bilderzeugungsapparate und entsprechender Verfahren zur Bilderzeugung oft Bilder, die in Operationen eingesetzt werden, in der Regel nicht aktuell. Das gilt zum Beispiel für präoperative Bilder, seit deren Aufnahme sich das Gewebe und seine Funktionen bereits verändert haben können. Werden intraoperative Bilder verwendet, so ergeben sich insbesondere bei Verwendung von beweglichen Detektoren Probleme damit, dass hierfür vorbekannte Auswertesysteme nicht in der Lage sind, ein hochwertiges Bild zu garantieren. Es besteht hier zur Verbessemng der Bilderzeugung Bedarf an einer Qualitätskontrolle, insbesondere an einer Qualitätskontrolle bereits während der Erfassung der Detektordaten. Eine solche Qualitätskontrolle kann auch eine fortlaufende Qualitätskontrolle sein. Ferner ist zur Verbesserung der Bilderzeugung ein verbesserter Datensatz wünschenswert, was durch Anleiten zum Detektieren gesichert werden kann. Speziell bei beweglichen oder gar in der Hand tragbaren Detektoren stellt die Erfassung von Detektordaten, die prinzipiell zu jedem Zeitpunkt bei einer beliebigen Position und/oder Orientierung des Detektors aufgenommen werden können, eine Herausforderung dar. Zur Verbesserung der Bilderzeugung ist es ferner wünschenswert, vorhandene Information wie beispielsweise über anatomische Gegebenheiten, Detektoreigenschaften, andere Materialeigenschaften, die die Detektion beeinflussen, oder über Zwangsbedingungen zu verwenden. Zur Verbesserung der Bilderzeugung kann auch ein Verbessern des Registrierens der Bilder beitragen. Auch ein Verändern und Verbessern der Abbildungsvorschrift schon während des Detektionszeitraums kann die Bilderzeugung insgesamt verbessern.

Beispielhaft werden Mittel zum Verbessern der Bilderzeugung bereitgestellt.

Sammeln von Detektordaten

Gemäß einem Aspekt der vorliegenden Erfindung werden Detektordaten von dem Auswertesystem gesammelt. Hierbei wurde eine Position und/oder Orientierung des Detektors von einem Erfassungssystem erfasst. Die Detektordaten umfassen Informationen über die detektierte radioaktive Strahlung. Weiter umfassen die Detektordaten Informationen über die Position und/oder Orientierung des Detektors. Beispielsweise können Daten mit Informationen über die detektierte Strahlung synchronisiert mit Daten über die Position und/oder Orientierung des Detektors gesammelt werden. Zur Synchronisation von Daten siehe WO 2007/131561, insbesondere Seite 3, Zeilen 1 bis 6 und Zeilen 27 bis 33, und Seite 6, Zeilen 22 bis 30, hier eingebunden durch Referenz. In weiteren Ausführungsformen werden die Detektordaten in dem Auswertesystem gespeichert.

Gemäß einem weiteren Aspekt der Erfindung detektiert ein Detektor Strahlung während eines Detektionszeitraums. Diese Strahlung ist radioaktive, bzw. Kernstrahlung. Unter solcher radioaktiver Strahlung wird auch Strahlung verstanden, die mittelbar durch radioaktive Zerfälle erzeugt wird, beispielsweise Ionisationsstrahlung eines Alpha-Teilchens. Ausführungsformen der Erfindung, in denen ein Detektor radioaktive Strahlung misst, umfassen daher auch das Detektieren solcher sekundären Strahlung.

Bilderzeugung und Bilderzeugungsvorschrift

In weiteren Ausführungsformen erzeugt das Auswertesystem aus den Detektordaten durch eine Bilderzeugungsvorschrift ein Bild. In typischen Ausführungsformen ist dieses Bild ein Bild der Strahlungsverteilung und damit der Strahlungsquellen in einem räumlichen Gebiet.

Beispielhaft ist die Bilderzeugungsvorschrift eine lineare Vorschrift. Hierbei wird typischerweise eine Abbildungsmatrix H, auch Systemmatrix genannt, auf einen Vektor $f=(f_1, f_2, ..., f_N)$ angewendet. Der Vektor f enthält Bildinfoimationen. Typischerweise wird zur Darstellung eines Bildes eines räumlichen Gebietes dieses räumliche Gebiet in Bildelemente (Voxel) aufgeteilt. Jeder Index $i$=1, 2, .... N des Vektors $f$ ist dann einem bestimmten Bildelement zugeordnet. Informationen bezüglich dieser Bildelemente (z.B. Strahlungsintensität in dem jeweiligen Bildelement) bilden die Einträge $f_i$ des Vektors f zu dem entsprechenden Index $i$.

Die Detektordaten werden ebenfalls in einem Vektor $g = (g_1, g_2, ...)$ zusammengefasst. Jeder Index $k$=1, ..., M ist dabei einer Messung (oder gemittelten Messreihe, s.u.) eines Detektors zugeordnet, und der Eintrag $g_k$ enthält das Ergebnis der bei dieser Messung gemessenen Strahlungsintensität.

Die Einträge $H_{ki}$ der Abbildungsmatrix H modellieren den Einfluss einer normierten Strahlungsquelle an der dem Index i zugeordneten Position auf die k-te Messung. Die Abbildungsmatrix H enthält in ihren Einträgen $H_{ki}$ Informationen über Positionen und Orientierungen des Detektors für radioaktive Strahlung. Da sich die verschiedenen Beiträge linear überlagern, ist bei einer Strahlungsverteilung $f_i$ ein Ergebnis der Messung zu erwarten, das in etwa durch den Vektor $g\_prognostiziert_k$ = Summe$_i$ $H_{ki}$ $f_i$ gegeben ist. In Matrix-Schreibweise (mit "*" als Matrix-Produkt):

$$g\_prognostiziert = H*f$$

Ein solcher Vektor $g\_prognostiziert$ kann mit einem Vektor $g\_gemessen$ verglichen werden, der tatsächliche Detektordaten mit Information über detektierte Strahlung beinhaltet. Bei diesem Vergleich sind selbstverständlich verschiedene Messfehler, z.B. Beiträge externer Strahlungsquellen, Unzulänglichkeiten des Detektors, statistische Fehler, etc. zu berücksichtigen.

Die Bilderzeugung kann nun derart beschrieben werden, einen Vektor f mit Bildinformation bezüglich der Strahlungsverteilung in einem räumlichen Gebiet zu finden, der mit den tatsächlich gemessenen Daten über nukleare Strahlung am besten in Einklang steht. Ein konzeptioneller Ansatz hierfür ist eine Minimierung des Abstands

$$|H*f - g\_gemessen|,$$

über alle geschätzten Strahlungsveiieilungen, deren jeweilige Bildinformation in einem jeweiligen Vektor f codiert ist. Hierbei bezeichnet |•| eine geeignete Abstandsnorm. In typischen Ausführungsformen wird |•| durch die $L_2$-Norm berechnet. Diese Minimierung kann als iterativer Prozeß implementiert sein. Der involvierte Minimierungsprozess kann beispielsweise durch algebraische Rekonstruktionstechniken, Maximum Likelihood Erwartungswertmaximierung, Pseudoinversion mittels Singulärwertdekomposition, Gauss-Seidel Inversion, sukzessive Überrelaxierung, Jacobi Inversion, multiplikative algebraische Rekonstruktionstechniken, simultane iterative Rekonstruktionstechniken oder durch andere Techniken ausgeführt werden. Auch Regularisierungsmethoden wie Tikhonov Regularisierung, Gesamtvariationsregularisierung und andere Regularisierungen können verwendet werden. Vor diesem Hintergrund ist die Bilderzeugungsvorschrift so definiert, dass sie in erster Linie durch die Matrix H gebildet wird. Aber auch der zu benutzende Algorithmus zum Lösen des Minimierungsproblems sowie der zu verwendende Startvektor einer iterativen Lösung sind Teil der Bilderzeugungsvorschrift.

Beispielhaft ist die Bilderzeugungsvorschrift nicht linear. Auch für solche nicht-linearen Bilderzeugungsvorschriften können analoge Verfahren angewendet werden.

Detektionsmodelle

Beispielhaft können Bilderzeugungsvorschliften, insbesondere die oben beschriebene Matrix H, aufgrund mindestens eines Detektionsmodells erzeugt oder verbessert werden. Detektionsmodelle können verändert oder angepasst werden, insbesondere aufgrund neuer Detektordaten. Detektionsmodelle können gemäß einigen Ausführungsformen verbessert oder fortlaufend verbessert werden. Verbesserte oder fortlaufend verbesserte Detektionsmodelle können zur Verbesserung einer Bilderzeugungsvorschrift verwendet werden.

Bei einer linearen Bilderzeugungsvorschrift können die Einträge der Abbildungsmatrix durch Detektionsmodelle berechnet werden. Solche Detektionsmodelle können durch algebraische, analytische, numerische oder statistische Methoden erstellt sein oder aufgrund von Messdaten erstellt sein oder durch Kombination davon. Beispielhaft werden Detektionsmodelle durch Messung an einer radioaktiven Punktquelle erstellt, die verschieden positioniert wird und deren Strahlung aus verschiedenen Positionen und Orientierungen vermessen wird. Durch solche Messungen oder durch geeignete Detektionsmodelle wird Information beispielsweise über mindestens eine Materialeigenschaft mindestens eines Materials gewonnen bzw. genutzt. Im Fall einer Bilderzeugung für medizinische Zwecke können beispielsweise Materialeigenschaften von im Raum verteilten Materialien wie Operationstisch, Gerätschaften, aber auch der Patient selbst bestimmt werden.

Materialeigenschaften umfassen die Abschwächung zwischen Quelle und Detektor, und eine Streuung zwischen Quelle und Detektor, die Materialeigenschaften von Materialien zwischen der Quelle und dem Detektor, die Abschwächung durch einen Detektorschild oder ein Streuen durch ein Detektorschild, die Abschwächung im Detektor selbst und die Streuung im Detektor selbst.

Weiter können sowohl analytische als auch algebraische, numerische statistische oder Kombinationsdetektionsmodelle neben Materialeigenschaften auch Zwangsbedingungen berücksichtigen. Beispiele für Zwangsbedingungen sind der relative Raumwinkel zwischen einem Detektor und einem Quellbereich von Strahlung, die Abmessungen des Detektors, oder aber ein Nichtvorhandensein von Material oder Materie. Zwangsbedingungen erlauben, gewisse Bildvektoren f von vorneherein auszuschließen und dadurch bessere Ergebnisse des oben beschriebenen Minimierungsproblems zu erreichen.

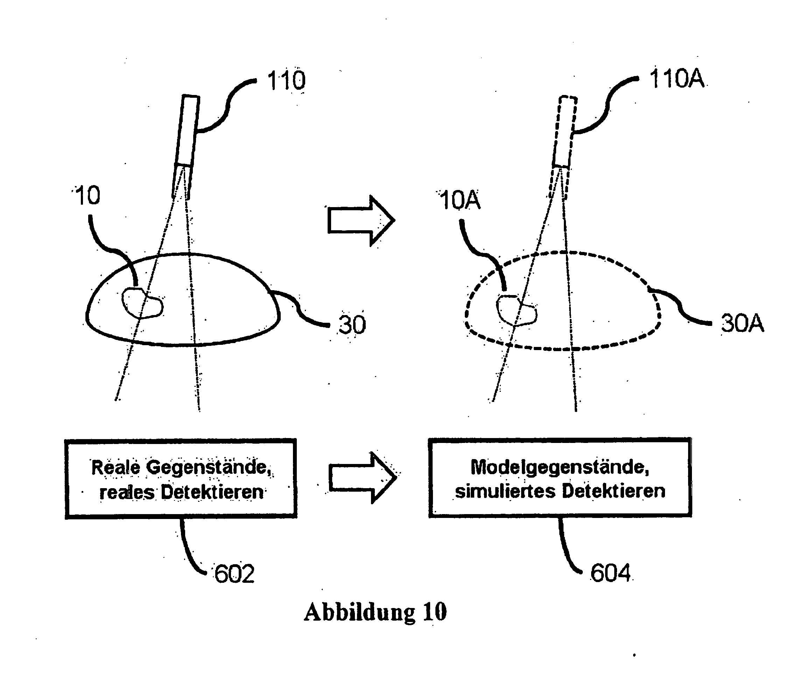

Abbildung 10 zeigt schematisch die Übersetzung von realen Objekten und einem realen Detektionsprozess in ein Detektionsmodell und einen simulierten Detektionsprozess. Gemäß Ausführungsformen der Erfindung werden reale Objekte wie ein Detektor 110, ein Körper 30 und eine sich innerhalb des Körpers befindliche Strahlungsquelle 10 auf Daten eines Detektionsmodells abgebildet. Dabei beschreiben Daten bezüglich des Detektors einen virtuellen Detektor 110a, Daten bezüglich des Körpers einen virtuellen Körper 30a und Daten bezüglich der Strahlungsquelle eine virtuelle Strahlungsquelle 10a.

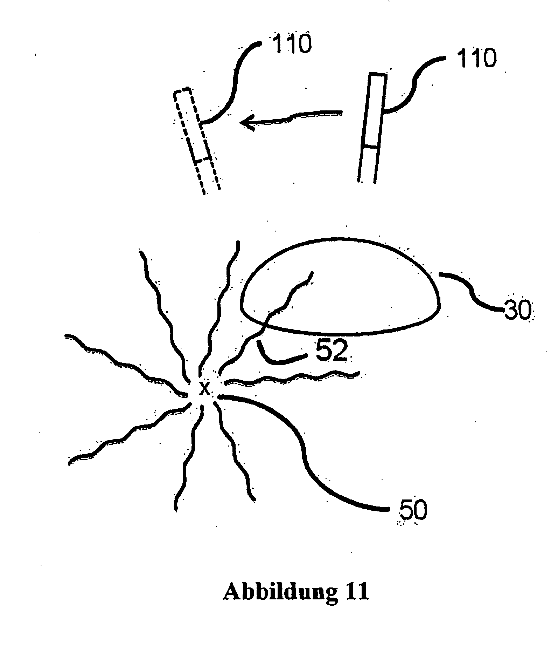

Abbildung 11 illustriert die Vermittlung eines Detektionsmodells aufgrund von Messungen. Eine radioaktive Punktquelle 50 emittiert radioaktive Strahlung 52 in alle Raumrichtungen. Ein Detektor 110 vermisst an verschiedenen Positionen und mit verschiedenen Orientierungen (zweite Position/Orientierung gestrichelt dargestellt) die Strahlungsquelle 50, wodurch Informationen über Materialeigenschaften gewonnen werden. Materialeigenschaften können beispielsweise diejenigen eines Körpers 30 umfassen. Aus den Messdaten kann ein Detektionsmodell gewonnen werden. Das Detektionsmodell kann die Information der Messdaten und weiter Information wie beispielsweise die Detektorgeometrie berücksichtigen.

Beispielhaft berücksichtigt das Detektionsmodell mindestens eine weitere Materialeigenschaft und/oder mindestens eine weitere Zwangsbedingung.

Materialeigenschaften können das Detektionsmodell beispielsweise aufgrund folgender Effekte beeinflussen: Abschwächung von Strahlung, Streuung von Strahlung, Beugung von Strahlung, Brechen von Strahlung, Einfluss von elektromagnetischen Feldern, Einfluss von Hintergrundstrahlung, Signalrauschen oder Einfluss von Fehlern in den Messungswerten des Detektors sowie in der Messung von Position und/oder Orientierung des Detektors.

Beispielhaft kann auch mindestens eine Zwangsbedingung berücksichtigt werden, wobei die Zwangsbedingung 15 beispielsweise der relative Raumwinkel zwischen dem Detektor und dem Quellbereich der Strahlung, die Abmessung des Detektors oder ein Nichtvorhandensein eines Materials sein können.

Beispielhaft berücksichtigt das Detektionsmodell eine Abschwächung von Strahlung, eine Streuung von Strahlung, eine Beugung von Strahlung, eine Brechung von Strahlung, den Einfluss von elektromagnetischen Feldern, den Einfluss von Hintergrundstrahlung, ein Signalrauschen, den Einfluss von Fehlern in den 15 Messungswerten des Detektors sowie in der Messung von Position und/oder Orientierung des Detektors oder weitere Effekte. Beispielhaft berücksichtigt das Detektionsmodell Zwangsbedingungen wie den relativen Raumwinkel zwischen dem Detektor und einem Quellbereich der Strahlung, die Abmessung des Detektors oder ein Nichtvorhandensein eines Materials oder Kombinationen dieser Zwangsbedingung.

Beispielhaft werden Bilderzeugungsvorschriften verändert. Insbesondere werden bei linearen Bilderzeugungsvorschriften die Einträge der Abbildungsmatrix oder Systemmatrix verändert. Beispielhaft wird die Systemmatrix verändert, sobald weitere Messdaten zur Verfügung stehen. Speziell kann die Minimierung der Norm der Differenz zwischen H angewendet auf $f$ und $g_{gemessen}$ erneut minimiert werden, sobald weitere Messdaten zur Verfügung stehen. Folglich umfassen Ausführungsformen typischerweise ein fortlaufendes Verändern der Bilderzeugungsvorschrift. Auch die Detektionsmodelle können fortlaufend angepasst und verbessert werden.

Registrierung

Beispielhaft werden Detektordaten mit kompatiblen Daten registriert. In einigen Ausführungsformen sind die kompatiblen Daten durch eine Abbildungsvorschrift aus einem vorgegebenen Bild gewonnen. Ein solches vorgegebenes Bild kann beispielsweise ein zuvor erfasstes (präoperativ aufgenommenes) anatomisches oder körperfunktionelles Bild sein. Im Falle einer linearen Abbildungsvorschrift kann diese durch eine Abbildungsmatrix H dargestellt werden wie oben beschrieben. Diese Matrix H kann von einem Lagevektor $T$ abhängen, in welchem Informationen über die relative Lage und/oder Orientierung zwischen dem Detektor und der Quelle der Strahlung erfasst ist. Hierbei kann $T$ eine relative Lage im Sinne einer rigiden Registrierung, oder im Sinne einer deformierbaren Registrierung beschreiben. Die Matrix $H(T)$, d.h. abhängend von $T$, wird auf einen Vektor $f_{Bild}$ angewendet, wie oben beschrieben, um einen Vektor mit dem Bild zugeordneten (theoretischen) Detektordaten $g = H(T) * f_{Bild}$ zu erhalten.

Die in $g$ enthaltene Information stellt prognostizierte oder virtuelle oder simulierte Detektordaten dar, die Simulationsdetektordaten genannt werden. Wie zuvor enthält ein Vektor $g_{gemessen}$ Information über detektierte Strahlung. Das Format (d.h. die Struktur des Vektors $g$) der Simulationsdetektordaten ist mit dem der gemessenen Detektordaten $g_{gemessen}$ kompatibel. Ein Registrieren von Detektordaten mit solchen kompatiblen Daten findet statt, gemäß einigen Ausführungsformen der Erfindung, indem der Abstand $|H(T)*f_{Bild} - g_{gemessen}|$ minimiert wird, d.h. zwischen $g$ und $g_{gemessen}$. Der Abstand $|\cdot|$ kann beispielsweise durch die $L_2$-Norm gegeben sein. Die Minimierung findet über alle Lage-Vektoren $T$ statt, um als Ergebnis der Minimierung einen optimalen Lage-Vektor $T$ zu erhalten. Bei Verwendung dieses optimalen Lage-Vektors $T$ wird durch die Matrix $H(T)$ den gemessenen Detektordaten ein Bildvektor zugeordnet, das mit dem Bildvektor des vorgegebenen Bildes kompatibel und registriert ist.

Beispielhaft wird die Minimierung durch Algorithmen wie beispielsweise den Best-Neighbour-Ansatz, einen Simplex-Optimierer, den Levenberg-Marquardt Algorithmus, den steilsten Gradientenabstieg, den konjugierten Gradientenabstieg oder andere ausgeführt.

Die Registrierung kann jedoch nicht nur durch Vergleich der Detektordaten $g$ wie oben beschrieben, sondern auch durch direkten Vergleich der aus den Detektordaten gewonnenen Bilddaten $f$ mit dem vorgegebenen Bild erfolgen. Dieser Vergleich kann etwa durch einen Bildvergleich mit den oben zu $g$ beschriebenen Methoden, oder etwa durch einen Vergleich einzelner hierfür vorgesehener Markierungspunkte erfolgen. Weiter sind auch andere Registrierungsmethoden möglich.

Der oben beschriebene Bildvergleich erlaubt es ferner, eine Abschätzung der Qualität von gesammelten Daten zu erreichen (als der Abweichung zwischen den aus den Detektordaten gewonnenen Bilddaten von dem vorgegebenen Bild).

Daten können mit kompatiblen Daten auch indirekt registriert werden. Unter indirekter Registrierung versteht sich ein Registrieren eines ersten Datensatzes mit einem dritten Datensatz veimittels eines zweiten Datensatzes. Hierfür wird zunächst der erste Datensatz mit dem zweiten Datensatz registriert, z.B. wie oben beschrieben. Dann wird der zweite Datensatz mit dem dritten Datensatz registriert. Unter Verwendung dieser Registrierung wird schließlich der erste mit dem dritten Datensatz registriert. Beispielsweise kann der erste Datensatz aus einem Basisbild wie zum Beispiel einem präoperativ aufgenommenen anatomischen Bild gewonnen worden sein. Der zweite Datensatz kann beispielsweise Detektordaten zu einem ersten Zeitpunkt entsprechen und der dritte Datensatz Detektordaten zu einem späteren Zeitpunkt. Ist die Registrierung des ersten Datensatzes, der aus dem Basisbild hergeleitet worden ist, mit dem zweiten Datensatz gelungen, erleichtert die Ähnlichkeit des zweiten und dritten Datensatzes aus Detektordaten eine Registrierung, wenn indirekte Registrierung verwendet wird wie oben beschrieben.

## Qualitätskontrolle

Um hochwertige, insbesondere aktuelle, hochwertige Bilder erzeugen zu können, stellen Aspekte der Erfindung Verfahren und Geräte zur Kontrolle der Qualität sowohl der Detektordaten als auch der erzeugten Bilder bereit. In einigen Ausführungsformen geschieht eine Qualitätskontrolle fortlaufend. Auf diese Weise wird die Güte und Gültigkeit eines erzeugten Bildes überprüft. In weiteren Ausführungsformen geschieht die Qualitätskontrolle bereits während des Detektionszeitraums.

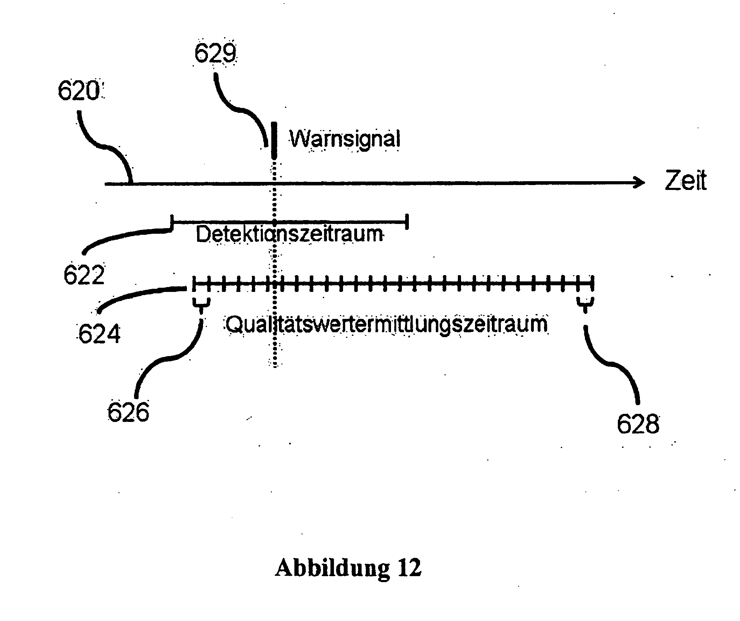

Abbildung 12 zeigt einen typischen Prozess der Qualitätskontrolle gemäß Ausführungsformen der Erfindung. Dargestellt ist eine Zeitachse 620, die den Verlauf der Zeit (von linksnach rechts) symbolisiert. In Abbildung 12 ist weiter ein Detektionszeitraum 622 gezeigt. Ferner ist, bezogen auf die gleiche Zeitachse, ein Qualitätswertermittlungszeitraum 624 abgebildet. Typischerweise beginnt der Qualitätswertermittlungszeitraum 624 nach dem Beginn des Detektionszeitraums, wenn bereits Detektordaten verfügbar sind. Der Qualitätswertermittlungszeitraum 624 kann vor dem, gleichzeitig mit dem oder nach dem Detektionszeitraum enden. Typischerweise endet der Qualitätswertermittlungszeitraum 624 nach dem Detektionszeitraum. Die Abstände zwischen Markierungen auf der Strecke, die den Qualitätswertermittlungszeitraum 624 symbolisiert, symbolisieren ihrerseits Zeiträume, in denen ein Qualitätsermittlungsprozess wie beispielsweise das Ermitteln eines Qualitätswerts durch die Auswerteeinheit stattfindet. Die Abstände 626 und 628 symbolisieren den ersten, bzw. letzten Qualitätsermittlungsprozess. In weiteren Ausführungsformen der Erfindung wird ein Warnsignal 629 ausgegeben, wenn erfasste, bzw. von der Auswerteeinheit gesammelte Daten eine Qualitätskontrolle nicht bestehen. Ein solches Warnsignal kann beispielsweise akustisch, optisch, haptisch oder durch Kombination davon ausgegeben werden. Ein solches Warnsignal kann einem Nutzer, beispielsweise einem Operierenden, vergegenwärtigen, dass Bilder, die aus den Detektordaten erzeugt werden, zumindest zum Zeitpunkt der Ausgabe des warnsignal möglicherweise nicht vertrauenswürdig sind.

Die Qualitätskontrolle findet anhand von mindestens einem Qualitätskriterium statt. Bezüglich eines oder mehrerer Qualitätskriterien wird ein Qualitätswert berechnet. Es können auch mehrere Qualitätswerte zu einem, bzw. mehreren Qualitätskriterien berechnet werden, beispielsweise wenn ein von einer jeweiligen Bildregion abhängiger Qualitätswert ermittelt wird. Hierbei kann beispielsweise die Gültigkeit oder Güte eines Bildes zurückgewiesen werden, wenn ein solcher Qualitätswert eines oder mehrere Qualitätskriterien nicht eifüllt, d.h. diesen nicht genügt. Andersherum kann ein Bild als gültig anerkannt werden, wenn ein Qualitätswert einem Qualitätskriterium genügt oder mehreren Qualitätskriterien genügt. Hier und im Folgenden kann statt eines Bildes auch eine bestimmte, dem jeweiligen Qualitätswert zugeordnete Bildregion verstanden werden.

Beispiele für Qualitätskriterien sind folgende: die Ähnlichkeit zwischen einem ersten Bild und einem zweiten Bild, wobei eines der Bilder oder beide Bilder aus Detektordaten erzeugt sein können, die Konditionierung einer Bilderzeugungsvorschrift zur Erzeugung eines Bildes, die Relevanz von Daten wie beispielsweise Detektordaten für ein Bildelement, die Plausibilität der Bilderzeugung aus Daten wie Detektordaten oder anhand des zweiten Bildes, die Uniformität von Daten wie Detektordaten, oder das Falscherzeugungsrisiko aufgrund von fehlerhaften Daten wie Detektordaten.

Die Ähnlichkeit zwischen einem ersten und einem zweiten Bild kann ähnlich bestimmt werden wie im Fall der Registrierung. Insbesondere können auch bereits registrierte Bilder wiederum miteinander auf Ähnlichkeit verglichen werden. Die Bilder können hierbei durch direkte Bildregistrierung oder durch Datenregistrierung registriert worden sein. Die Bilder können beispielsweise anatomische oder organfunktionelle Bilder sein, wie die oben bereits beschriebenen oder weitere.

Ist die Bilderzeugungsvorschrift eine lineare Vorschrift, so kann die Konditionierung einer Bilderzeugungsvorschiift beispielsweise durch die Konditionierung der Abbildunasmatiix oder der Systemmatrix gegeben sein. Insbesondere kann in einem linearen, diskreten Fall die Konditionszahl der Abbildungsmatrix H (siehe oben) berechnet werden. Die Konditionszahl kann durch Analyse des Spektrums der Singulärwerte der Matrix oder durch ähnliche Matrixdekompositionsmaße berechnet werden (z.B. Verhältnis von größtem zu kleinstem Eigenwert, oder Anzahl der einen Schwellwert über- oder unterschreitenden Eigenwerte). In diesem Beispiel ist das Qualitätskriterium ein Schwellenwert für die Konditionierungszahl. Ist die berechnete Konditionierungszahl, d.h. der Qualitätswert, kleiner (bzw. größer, je nach Definition der Konditionierungszahl) als dieser Schwellenwert, so eifüllen die Daten wie beispielsweise Detektordaten das Qualitätskriterium nicht, und folglich wird ein daraus erzeugtes Bild zurückgewiesen. Ist auf der anderen Seite die berechnete Konditionierungszahl größer (bzw. kleiner) als der Schwellenwert, so wird die Güte der Daten wie zum Beispiel Detektordaten und ein daraus rekonstruiertes Bild akzeptiert.

Ähnlich kann die mit dem englischen Fachausdruck *Sparsity* (Dünnbesetztheit) einer Matrixzeile oder -spalte bezeichnete Größe als Qualitätswert, und ein Schwellenwert bezüglich dieser Größe als Qualitätskriterium verwendet werden. Eine Zeile oder eine Spalte einer Matrix ist dünn besetzt *(sparse),* wenn weniger als eine durch den Schwellenwert festgelegte Zahl von Einträgen von Null (bzw. numerisch Null, d,h. kleiner als ein vorgegebener epsilon-Schwellwert) verschieden ist. Ist eine Matrixspalte zu dünn besetzt, hängt ein Bildelement von nur wenigen Messungen ab, und es besteht für dieses Bildelement daher ein hohes Falscherzeugungsrisiko. Ist eine Matrixzeile zu dünn besetzt, so ist der dieser Zeile zugeordnete Messwert für nur wenige Bildelemente verantwortlich, d.h. hat umgekehrt ein übermäßig hohes Gewicht für diese Bildelemente, was wiederum ein hohes Falscheizeugungsrisiko mit sich bringen kann.

Entsprechend kann auch die Relevanz von Daten für ein Bildelement als Qualitätskriterium verwendet werden. Für eine lineare Bilderzeugungsvorschrift kann beispielsweise die Relevanz einer Zeile oder Spalte mit einem Schwellenwert für die Summe aller Einträge einer Zeile oder Spalte verknüpft werden.

Die Plausibilität einer Bilderzeugung berücksichtigt beispielsweise eine Zwangsbedingung. Beispiele für Zwangsbedingungen sind die Maximalmenge von Strahlung, der Gradient der Summe der Maximalstrahlung, minimale Strahlung, Strahlung, die in Bildelementen zuzuordnen ist, welche offensichtlich keine Strahlungsquellen enthalten können (zum Beispiel luftgefüllte Volumina), und weitere. Je nach Grad der Plausibilität kann ein entsprechender Qualitätswert zugeordnet werden.

Die Uniformität von Detektordaten wird durch die räumliche Verteilung der Messungen bestimmt. Uniforme Messungen liegen vor, wenn die Messungen gleichmäßig um das zu rekonstruierende Gebiet verteilt sind. Ein Maß für die Uniformität wird durch die Abweichung der tatsächlichen Messungen von einer vollkommen uniformen Messung gebildet. Das Qualitätskriterium ist durch einen Schwellenwert bezüglich dieser Uniformität gebildes.

Beispielhaft wird eine Qualitätskontrolle basierend auf den oben genannten oder anderen Qualitätskriterien fortlaufend, vorzugsweise quasi-kontinuierlich ausgeführt (wie in Abb. 12 dargestellt). In weiteren Ausführungsformen wird das Ergebnis der Qualitätskontrolle durch das Ausgabesystem an einen Nutzer ausgegeben. Insbesondere kann die Ausgabe wie oben beschrieben visuell, akustisch oder haptisch erfolgen. Beispielsweise kann die Ausgabe durch eine Vergröberung der Bildauflösung an der entsprechenden Bildregion erfolgen. Dadurch wird der Nutzer vor falschem Vertrauen in möglicherweise fehlerhafte Bilder bewahrt. Beispielhaft wird der mindestens eine Qualitätswert bezüglich mindestens eines Qualitätskriteriums ermittelt. Ein Qualitätskriterium kann beispielsweise die Ähnlichkeit zwischen einem aus den gesammelten Detektordaten erzeugten ersten Bild und einem zweiten Bild sein, die Konditionierung einer Bilderzeugungsvorschrift zur Erzeugung eines Bildes aus den gesammelten Detektordaten, die Relevanz der gesammelten Detektordaten für ein Bildelement, die Plausibilität einer Bilderzeugung aus den gesammelten 10 Detektordaten, die Uniformität der gesammelten Detektordaten, oder das Falscherzeugungsrisiko aufgrund von fehlerhaften Detektordaten. Daneben können weitere Qualitätskriterien verwendet werden.

### Verbesserung der Bilderzeugung

Ausführungsformen der Erfindung entsprechend werden Verfahren und Apparate zur Bilderzeugung bereitgestellt, bei denen die Qualität der Bilderzeugung verbessert wird. In typischen Ausführungsformen wird die Qualität fortlaufend verbessert. Insbesondere kann die Qualität während des Detektionszeitraums bereits verbessert werden oder fortlaufend verbessert werden.

Beispielhaft erfolgt die Bilderzeugung aufgrund einer linearen Bilderzeugungsvorschrift. Diese kann beispielsweise durch die Anwendung einer Abbildungsmatrix oder Systemmatrix H auf einen Vektor $f$ erfolgen, wobei H und $f$ die oben erklärten Bedeutungen haben. Die Bilderzeugung kann, wie oben beschrieben, durch Vergleich des Ergebnisvektors $g$ = H*$f$ mit dem Detektordaten-Vektor $g_{gemessen}$ erfolgen (bzw. durch äquivalente Verfahren). Die Bilderzeugung, auch Rekonstruktion genannt, erfolgt wie oben beschrieben durch Minimierung des Abstands von Vektor $g$ und Vektor $g_{gemessen}$ als Funktion von $f$.

Eine Verbesserung der Bilderzeugung kann auf verschiedene Arten geschehen, welche umfassen: Verbessern des Anfangswertes von Vektor $f$ im Minimierungsproblem, Verbessern der Bilderzeugungsvorschift, insbesondere der Abbildungsmatrix H.

Als Anfangswert für das Minimierungsproblem kann beispielsweise ein Vektor $f_{Anfang}$ verwendet werden, dessen enthaltene Information aus einem vorgegebenen Bild abgeleitet ist, zum Beispiel aus einem präoperativen anatomischen oder organfunktionellen Bild. Dies hilft zu vermeiden, bei der Lösung des Minimierungsproblems eine falsche Lösung zu erhalten (etwa ein lokales Minimum, das nicht der gewünschten Lösung entspricht). Auch kann die Berechnungszeit verringert werden, da bereits mit einer annähernd korrekten Lösung begonnen wird. Somit kann eine gute Lösung des Minimierungsproblems, d.h. ein gutes Bild f, mit vermindertem Aufwand erhalten werden.

Eine Verbesserung der Abbildungsvorschrift bzw. der Abbildungsmatrix H kann insbesondere unter Berechnen von mindestens einem Qualitätswert erfolgen, wobei der Qualitätswert derselbe wie bei der Qualitätskontrolle der Daten beschrieben (s.o.) oder ein weiterer Qualitätswert sein kann. Zusätzlich wird die Abbildungsmatrix H unter Berücksichtigung des Qualitätswerts verändert. Insbesondere wird die Abbildungsmatrix H so verändert, dass die veränderte Matrix H einem oder mehreren Qualitätskliterien besser genügt.

Beispielsweise können Zeilen oder Spalten, die gemäß des Schwellenwerts bezüglich der Sparsity einer Matrix als zu dünn besetzt erkannt wurden, eliminiert werden. Ebenso können Zeilen oder Spalten der Abbildungsmatrix H eliminiert werden, die dem Kriterium der Relevanz nicht genügt haben. Statt einer reinen Elimination können auch ähnliche Zeilen und Spalten kombiniert werden, wobei auch die zugehörigen Bildelemente (Einträge von $f$) bzw. Detektormesswerte (Einträge von $g$) entsprechend kombiniert werden.

Die Uniformität kann ferner beispielsweise verbessert werden, indem die Detektordaten benachbarter Messungen kombiniert werden, so dass eher uniform verteilte effektive Messungen erhalten werden. Durch derartige. Kombinationen wird die Abbildungsmatrix kleiner und auch aus diesem Grund wird die Rekonstruktion numerisch besser lösbar.

Durch diese Kombination können andererseits Informationen verloren gehen. Um den Informationsverlust zumindest teilweise zu kompensieren, kann den aus mehreren Werten gemittelten Einträgen ein erhöhtes Gewicht beigemessen werden, der ihrer höheren statistischen Signifikanz Rechnung zu tragen. Beispielsweise kann der Beitrag solcher Einträge zur Abstandsnorm |·| ein erhöhtes Gewicht erhalten.

Gemäß weiteren Ausführungsformen werden zur Verbesserung der Bilderzeugung folgende weitere Methoden verwendet:

### Verwendung von Oberflächeninformation

Wenn die Oberfläche des räumlichen Gebiets bekannt ist, das die Strahlungsquelle umfasst, können mögliche Abbildungen eliminiert werden, die Informationen über Bildelemente enthalten, die nicht innerhalb dieser Oberfläche liegen. Insbesondere kann diese Oberfläche beispielsweise die Körperoberfläche eines Patienten sein. Diese kann abgescannt werden durch Laserbereichsscanner, Laseroberflächenmusterscanner, Laserpointeroberflächenscanner, stereoskopische Kamerasysteme, Time-Of-Flight-Kameras und weitere Oberflächenerfassungssysteme.

Auch kann derartige Oberflächeninformation aufgrund der Geometrie eines durch das Erfassungssystem erfassten Objekts und seiner erfassten Trajektorie ermittelt sein: Falls das Objekt etwa nicht in das Patientengewebe eindringen kann, so müssen die durch das Objekt selbst überstrichenen Raumbereiche luftgefüllt sein und können daher keine Strahlungsquelle enthalten. Insbesondere kann das Objekt durch den Detektor selbst gebildet werden bzw. integral mit dem Detektor sein.

### Benutzung anatomischer Information

Wenn, etwa im Fall der medizinischen Bildgebung, die Anatomie in einem Gebiet des erzeugten Bildes bekannt ist, können Zwangsbedingungen aufgrund der Kenntnis der Anatomie gesetzt und berücksichtigt werden. Beispielsweise kann eine Zwangsbedingung sein, dass Körperteile wie Knochen oder die Luftröhre (die z.B. bei einem bestimmten Tracer keine radioaktiven Strahlenquellen darstellen können) keine Strahlungsaktivität aufweisen können. Auf diese Weise können mögliche Abbildungen eliminiert werden, die fälschlicherweise diesen Bereichen eine Strahlungsaktivität zuschreiben würden. Anatomische Information kann beispielsweise durch zuvor aufgenommene anatomische Bilder erhalten werden. Diese können mit aktuellen Daten registriert sein. Auch Standard-Daten etwa aus anatomischen Atlanten können verwendet werden, welche ebenfalls mit aktuellen erzeugten Bildern registriert sein können. Anatomische Information kann aber auch aktuell erhalten werden durch weitere Detektoren des Detektorsystems wie beispielsweise Ultraschallgeräte, Computertomografen, Radiografen, optische Kameras, Magnetresonanztomografiegeräte und weitere.

Benutzung weiterer Strahlungsdetektoren

Das Detektorsystem kann auch weitere Strahlungsdetektoren umfassen. Während Detektordaten können ebenfalls verwendet werden zur Bilderzeugung. Die weiteren Detektoren können Strahlungsdetektoren sein, insbesondere Strahlungsdetektoren für radioaktive Strahlung. Die weiteren Detektoren können bewegliche Strahlungsdetektoren sein. Die weitem Strahlungsdetektoren können auch fixierte Strahlungsdetektoren sein. Beispielsweise kann der Tisch, auf dem die Strahlungsverteilung liegt, eine Gammakamera enthalten. In weiteren Ausführungsformen werden Boden-, Decken-, und/oder Wanddetektoren verwendet.

Beispielhaft ist das Programm zum Ermitteln mindestens eines Qualitätswerts ein Programm zum Ermitteln mindestens eines Qualitätswerts bezüglich mindestens eines Qualitätskriteriums. Qualitätskriterien können die weiter oben im Abschnitt "Qualitätskontrolle" beschriebenen Qualitätskriterien oder andere sein.

Ausgeben einer Anweisung an einen Nutzer

Aspekte der vorliegenden Erfindung umfassen das Ausgeben einer Anweisung an einen Nutzer. Insbesondere umfassen Aspekte der Erfindung das Ausgeben an einer Anweisung an einen Nutzer zum weiteren Bewegen des Detektors in Abhängigkeit von bereits gesammelten Detektordaten. Typische Ausführungsformen umfassen ein fortlaufendes Anweisen zur Detektion aufgrund einer fortlaufenden Qualitätskontrolle, welche oben beschrieben wurde. Das Ausgeben erfolgt durch das Ausgabesystem, insbesondere in optischer, akustischer oder haptischer Form oder durch Kombination davon. Speziell werden Anweisungen zum weiteren Bewegen des Detektors gegeben, sodass bei Befolgen die Qualität der gesammelten Detektordaten verbessert wird. Es werden Anweisungen zum weiteren Bewegen des Detektors in Abhängigkeit von den gesammelten Detektordaten ausgegeben, so dass sich bei Befolgen die Qualität der Detektordaten voraussichtlich am stärksten verbessern wird. Die Anweisungen können beispielsweise als Ausgabe eines Pfeiles erfolgen, der in eine Richtung weist, in der weitere Messungen vorgenommen werden sollen.

Typischerweise geht dem Ausgeben einer Anweisung die Berechnung der aktuellen Qualität oder Güte oder Gültigkeit der gesammelten Detektordaten voran und eine Berechnung, wie sich die Qualität der Daten verändern würde, wenn weitere Detektordaten zur Verfügung ständen, insbesondere Detektordaten mit Information über die detektierte Strahlung gemessen aus verschiedenen Orientierungen und Positionen des Detektors.

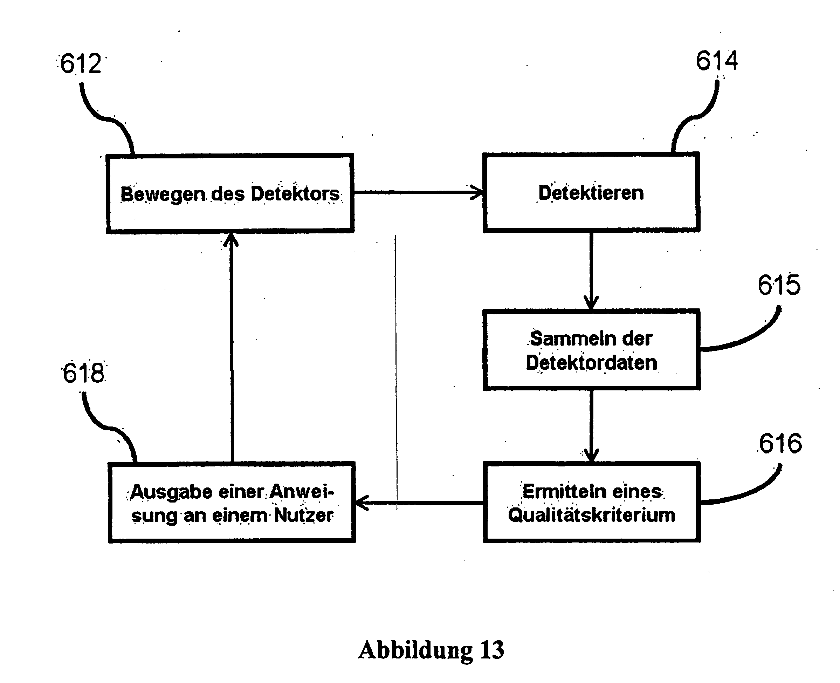

Abbildung 13 zeigt iterative Verfahrensschritte gemäß Ausführungsformen der Erfindung. Einer der iterativen Schritte ist ein Bewegen des Detektors. In typischen Ausführungsformen wird ein frei beweglicher, beispielsweise tragbarer Detektor verwendet. Nach oder während des Bewegens erfolgt ein Detektieren 614 von Strahlung durch den Detektor. Anschließend oder währenddessen erfolgt ein Sammeln 615 von Detektordaten mit Information über die detektieren Strahlung durch das Auswertesystem. Auch werden weitere Detektordaten wie Position und/oder Orientierung des Detektors gesammelt, in der Regel synchronisiert mit den Detektordaten mit Information über die detektiert Strahlung. Auf der Grundlage der Detektordaten findet ein Ermitteln 616 eines Qualitätskriteriums durch die Auswerteeinheit statt. Anschließend erfolgt eine Ausgabe 618 einer Anweisung an einen Nutzer. Ausführungsformen der Erfindung entsprechend weist die Ausgabe 618 einen Nutzer zum Bewegen des Detektors in einer Weise an, dass eine den Anweisungen entsprechende Bewegung zur nachfolgenden Erfassung von brauchbaren Detektordaten führt. Brauchbare Detektordaten sind typischerweise Detektordaten, die eine Bilderzeugung verbessern.

Typischerweise wird diejenige Position und Orientierung des Detektors, die die Qualität voraussichtlich am meisten verbessert, dem Nutzer ausgegeben. Eine Ausgabe, beispielsweise in akustischer Form, kann in Form eines sich intensivierenden Signallautes dargestellt werden. Eine Ausgabe in haptischer Form kann beispielsweise die Vermittlung eines Gefühls eines Widerstandes oder eines Gezogenwerdens sein. Diese Vermittlung kann z.B. durch mechanische Führung oder durch elektrische Stimulierung von Muskeln oder Gehirn erfolgen.

Zur Berechnung der Orientierung und Positionen, die die Abbildung voraussichtlich verbessern, können auch anatomische oder organfunktionelle Bilder verwendet werden.

Freihanderfassung

Intrinsische Probleme einer Verarbeitung von Detektordaten, die insbesondere bei einem frei beweglichen Detektor wie einem Freihanddetektor auftreten, entstehen dadurch, dass Messungen im Prinzip zu jedem Zeitpunkt und mit beliebiger Position und/oder Orientierung des Detektors erfolgen können. Dadurch können beispielsweise Daten aufgenommen werden, bei denen der Detektor überhaupt nicht auf die zu detektierende Strahlenquelle gerichtet ist. Ähnliche oder weitere Quellen für unbrauchbare Daten existieren. Solche Daten können die Bilderzeugung verschlechtern. Beispielsweise können solche Daten eine Abbildungsmatrix bezüglich Relevanz oder Sparsity verschlechtern.

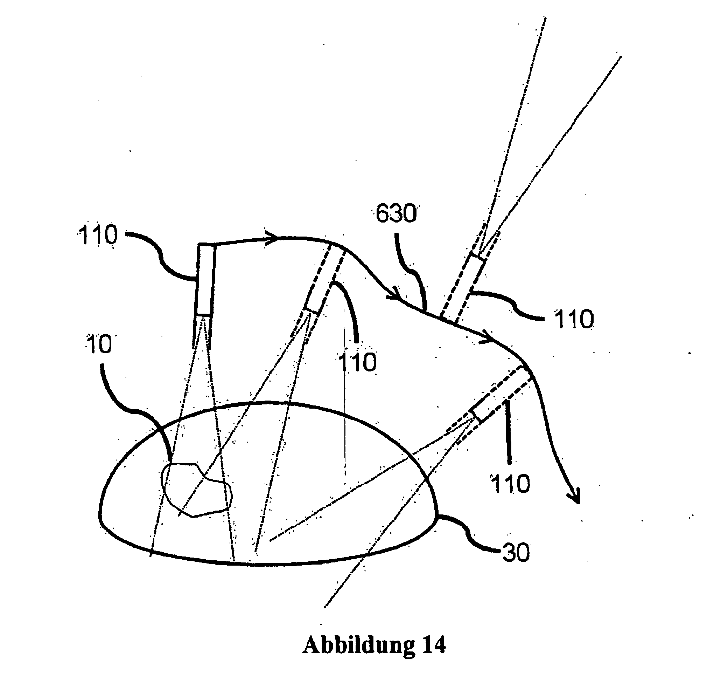

Abbildung 14 zeigt einen frei beweglichen Detektor 110, der entlang einer beliebigen Trajektorie bewegt wird. Die Bewegungsrichtung wird durch Pfeile entlang der Trajektorie gekennzeichnet. Positionen und Orientierungen, die auf die zeitlich erste Position und Orientierung folgen, sind gestrichelt dargestellt. Der Detektor 110 misst zu verschiedenen, im Allgemeinen beliebigen Zeitpunkten die Emissionen einer Strahlungsquelle 10 innerhalb eines räumlichen Gebiets 30. Die Strahlungsquelle 10 kann beispielsweise eine radioaktive Strahlungsverteilung sein im Körper eines Lebewesens. Abbildung 14 zeigt mindestens eine Position und Orientierung 630 des Detektors, die voraussichtlich zu unbrauchbaren Detektordaten hinsichtlich der gemessenen Strahlung führen. Unbrauchbare Detektordaten verschlechtern typischerweise die Bilderzeugung..

Aus diesem und weiteren Gründen benötigt eine Datenerfassung mit frei beweglichen Detektoren noch mehr als eine Detektion mit fixen oder beschränkt beweglichen Detektoren eine Qualitätskontrolle. Neben der Qualitätskontrolle kann eine Verbesserung der Bilderzeugungsvorschrift erfolgen.

Gemäß einem Aspekt der Erfindung findet eine Qualitätskontrolle und/oder eine aktive Verbesserung der Bilderzeugungsvorschrift bereits während des Detektionszeitraums statt im Gegensatz zu einer Nachauswahl, englisch "post selection". Beispeilhaft findet eine Qualitätskontrolle wiederholt oder fortlaufend statt, typischerweise quasi-kontinuierlich.

Ebenso kann eine Verbesserung der Abbildungsvorschrift wiederholt oder fortlaufend, typischerweise quasi-kontinuierlich stattfinden. Eine Verbesserung kann erfolgen wie beispielsweise im Abschnitt "Verbesserung der Bilderzeugung" beschrieben oder auf andere Art.

Gemäß weiteren Ausführungsformen der Erfindung, die mit anderen Ausführungsformen kombiniert werden können, ist das Verfahren zur Bilderzeugung ein Verfahren zur Bilderzeugung für medizinische Zwecke. Gemäß weiteren Ausführungsformen umfasst das Verfahren zur Bilderzeugung ein Sammeln von Körperdaten eines Lebewesens durch das Auswertesystem. Typischerweise umfassen die Körperdaten die Atemfrequenz und/oder die Herzfrequenz. Typischerweise umfassen die Körperdaten auch Daten bezüglich Form, Position und/oder Orientierung des Körpers. In weiteren typischen Ausführungsformen werden die Körperdaten bezüglich der Atemfrequenz und/oder der Herzfrequenz synchronisiert mit den Körperdaten bezüglich Form, Position und/oder Orientierung des Körpers gesammelt. Das Erfassen der Körperdaten des Lebewesens kann beispielsweise durch das Erfassungssystem erfolgen.

Beispielhaft umfasst das Verfahren zur Bilderzeugung weiter ein Verändern einer Bilderzeugungsvorschrift aufgrund der gesammelten Körperdaten. Insbesondere können dadurch Bewegungen des Körpers, beispielsweise durch Atmung oder Herzschlag, bei der Bilderzeugung berücksichtigt werden. Dies führt zu einer verbesserten Bilderzeugung. Auch das Registrieren von Bildern oder das Registrieren von Detektordaten wird dadurch erleichtert.

Beispielhaft umfasst das Verfahren zur Bilderzeugung ein Sammeln von Daten mindestens eines Instruments, vorzugsweise eines medizinischen Instruments, durch das Auswertesystem. Beispielhaft umfasst das Verfahren weiter ein Registrieren von Daten medizinischer Instrumente mit Detektordaten und/oder Simulationsdetektordaten durch das Auswertesystem. In typischen Ausführungsformen umfasst das Verfahren weiter ein Erzeugen eines Kombinationsbildes auf der Grundlage des Registrierens.

Gemäß weiteren Ausfühmngsformen umfasst das Verfahren weiter ein Erfassen von Daten medizinischer Instrumente durch das Erfassungssystem.

Beispielhaft umfasst das Verfahren ein Ausgeben des Kombinationsbildes durch das Ausgabesystem. Gemäß weiteren Ausführungsformen umfasst das Verfahren ein Anweisen eines Nutzers zur Benutzung der medizinischen Instrumente auf der Grundlage des Kombinationsbildes. Gemäß noch weiteren Ausführungsformen umfasst da Verfahren ein Führen des Nutzers beim Benutzen der medizinischen Instrumente durch ein Führungssystem auf der Grundlage der Instrumentendaten. Das Führungssystem kann eine Führungseinheit umfassen, die ein Nutzer auf haptische, akustische oder visuelle Weise führt oder durch Kombination davon.

Insbesondere kann das Anweisen eines Nutzers zum Benutzen der medizinischen Instrumente auf der Grundlage des Kombinationsbildes oder das Führen eines Nutzers beim Benutzen der medizinischen Instrumente durch ein Führungssystem beispielsweise durch Visualisierung einer virtuellen Realität, Visualisierung einer erweiterten Realität, Schicht- und Vielschichtvisualisierung, frequenzmodulierten Ton, amplitudenmodulierten Ton, pulsmodulierten Ton, durch Kombination derselben oder auf andere Weise erfolgen.

**Patentansprüche**

1. Verfahren zur Bilderzeugung durch einen Bilderzeugungsapparat (1), welches umfasst:

    - Detektieren (614) von radioaktiver Strahlung durch einen beweglichen Detektor (110) während eines Detektionszeitraums (622);
    - Sammeln (615) von Detektordaten zur Bilderzeugung durch ein Auswertesystem (300) des Bilderzeugungsapparats (1);
    wobei die Detektordaten Information über die detektierte Strahlung umfassen;

- wiederholtes Ermitteln (616) mindestens eines Qualitätswerts aus den gesammelten Detektordaten durch das Auswertesystem während des Detektionszeitraums; und

- Ausgeben (618) einer Anweisung an einen Nutzer zum weiteren Bewegen des Detektors (110) in Abhängigkeit von dem mindestens einen ermittelten Qualitätswert, wobei sich die Anweisung zumindest auf einen Teil des verbleibenden Detektionszeitraums (622) bezieht, **dadurch gekennzeichnet, dass** die Detektordaten Information über die Position und/oder Orientierung des Detektors umfassen.

2. Verfahren zur Bilderzeugung nach Anspruch 1, wobei der Detektor (110) frei beweglich ist, und typischerweise ein Freihanddetektor ist.

3. Verfahren zur Bilderzeugung nach einem der vorherigen Ansprüche, wobei das Verfahren weiter umfasst:

   fortlaufendes Sammeln von Detektordaten zur Bilderzeugung durch das Auswertesystem (300) des Bilderzeugungsapparats während des Detektionszeitraums (622).

4. Verfahren zur Bilderzeugung nach einem der vorherigen Ansprüche, wobei das Verfahren weiter umfasst:

   - Ausgeben des mindestens einen ermittelten Qualitätswerts an einen Nutzer.

5. Verfahren zur Bilderzeugung nach einem der vorherigen Ansprüche, wobei das Verfahren weiter umfasst:

   - Ausgeben einer Warnung an einen Nutzer, wenn der mindestens eine Qualitätswert einen Schwellwert nicht erreicht.

6. Verfahren zur Bilderzeugung nach einem der vorherigen Ansprüche, wobei das Ausgeben einer Anweisung an einen Nutzer zum weiteren Bewegen des Strahlungsdetektors das Ausgeben derjenigen Position und/oder Orientierung des Detektors (110) umfasst, deren Einnehmen durch den Detektor gemäß einer Prognose die Bilderzeugung gemäß mindestens einem Qualitätswert am stärksten verbessern würde.

7. Verfahren zur Bilderzeugung nach einem der vorherigen Ansprüche, wobei das Ausgeben der Anweisung an den Nutzer und/oder das Ausgeben des mindestens einen ermittelten Qualitätswerts auf einem Ausgabesystem mit mindestens einer Ausgabeeinheit visuell, akustisch, haptisch oder durch Kombinationen davon erfolgt.

8. Verfahren zur Bilderzeugung nach einem der vorherigen Ansprüche, wobei das Verfahren weiter umfasst:

   - Sammeln von Körperdaten durch das Auswertesystem (300) bezüglich Atmung und/oder Herzschlag des Lebewesens, welche insbesondere Atemfrequenz und/oder Herzfrequenz umfassen, und/oder von Körperdaten, die Form, Position und/oder Orientierung des Körpers umfassen,

   wobei optional die Körperdaten bezüglich Atmung und/oder Herzschlag mit den Körperdaten bezüglich Form, Position und/oder Orientierung des Körpers synchronisiert sind; und

   - Verändern einer Bilderzeugungsvorschrift aufgrund der gesammelten Körperdaten.

9. Verfahren zur Bilderzeugung nach einem der vorherigen Ansprüche, wobei das Verfahren weiter umfasst:

   - Erfassen von Daten medizinischer Instrumente (120) durch das Erfassungssystem, und typischerweise Erzeugen eines Instrumentenbildes auf der Grundlage der gesammelten Instrumentendaten durch das Auswertesystem; und

   - Führen des Nutzers beim Benutzen der medizinischen Instrumente durch ein Führungssystem auf der Grundlage der Instrumentendaten.

10. Bilderzeugungsapparat zur Bilderzeugung, welcher umfasst:

    - einen beweglichen Detektor (110) zur Detektion von radioaktiver Strahlung während eines Detektionszeitraums (622), und

    - ein Auswertesystem (300), welches umfasst:

      ein Schnittstellensystem (306) zur Übermittlung, an das Auswertesystem, von Detektordaten mit Information über die detektierte radioaktive Strahlung und über die Position und/oder Orientierung des Detektors zur Bilderzeugung,

      einen Datenspeicherabschnitt (320) zum Speichern der Detektordaten, und

      einen Programmspeicherabschnitt (330) mit einem Programm zum wiederholten Ermitteln mindestens eines Qualitätswerts hinsichtlich der Bilderzeugung aus den Detektordaten während des Detektionszeitraums; und wobei der Bilderzeugungsapparat weiter umfasst:

- ein Ausgabesystem (400), welches mindestens eine Ausgabeeinheit (410) umfasst,
wobei die mindestens eine Ausgabeeinheit (410) zum Ausgeben einer Anweisung an einen Nutzer zum weiteren Bewegen des Detektors in Abhängigkeit von dem mindestens einen ermittelten Qualitätswert umfasst,
wobei sich die Anweisung zumindest auf einen Teil des verbleibenden Detektionszeitrams (622) bezieht.

11. Bilderzeugungsapparat zur Bilderzeugung nach Anspruch 10,
wobei der Detektor (110) frei beweglich ist, und typischerweise ein Freihanddetektor ist.

12. Bilderzeugungsapparat zur Bilderzeugung nach einem der Ansprüche 10-11,
wobei das Schnittstellensystem (306) ein Schnittstellensystem zur fortlaufenden Übermittlung von Detektordaten an das Auswertesystem (300) mit Information über die detektierte Strahlung und mit Information über die Position und/oder Orientierung des Detektors (110) zur Bilderzeugung ist.

13. Bilderzeugungsapparat zur Bilderzeugung nach einem der Ansprüche 10-12,
wobei die mindestens eine Ausgabeeinheit (410) eine Ausgabeeinheit umfasst zum Ausgeben des mindestens einen ermittelten Qualitätswerts an einen Nutzer und/oder einer Warnung an einen Nutzer, wenn der mindestens eine Qualitätswert mindestens ein Qualitätskriterium nicht erfüllt.

14. Bilderzeugungsapparat zur Bilderzeugung nach einem der Ansprüche 10-13,
wobei die mindestens eine Ausgabeeinheit (410) eine Ausgabeeinheit umfasst zum Ausgeben derjenigen Position und/oder Orientierung des Detektors, deren Einnehmen durch den Detektor gemäß einer Prognose die Bilderzeugung gemäß mindestens einem Qualitätswert am stärksten verbessern würde.

15. Bilderzeugungsapparat (1) zur Bilderzeugung nach einem der Ansprüche 10-14, wobei der Bilderzeugungsapparat weiter umfasst:

- ein Erfassungssystem (200) zum Erfassen von Detektordaten über Position und/oder Orientierung des Detektors,
wobei das Erfassungssystem (200) weiter umfasst:
- mindestens einen Sensor zum Erfassen von Körperdaten bezüglich Atmung und/oder Herzschlag eines Lebewesens, welche insbesondere Atemfrequenz und/oder Herzfrequenz umfassen, und/oder
- eine Erfassungseinheit zum Erfassen von Körperdaten des Lebewesens, wobei die Daten Form, Position und/oder Orientierung des Körpers umfassen,
und wobei das Auswertesystem weiter umfasst:
- optional einen Programmspeicherabschnitt mit einem Programm zum synchronisierten Sammeln von Körperdaten des Lebewesens und
- einen Programmspeicherabschnitt mit einem Programm zum Verändern einer Bilderzeugungsvorschrift aufgrund der gesammelten Körperdaten.

**Claims**

1. A method of image generation by an image generating apparatus (1), comprising:

    - detecting (614) radioactive radiation by means of a movable detector (110) during a detection period (622);
    - collecting (615) detector data for image generation by means of an evaluating system (300) of the image generating apparatus (1);
    wherein the detector data contain information about the detected radiation;
    - repeatedly determining (616) at least one quality value from the collected detector data by the evaluating system during the detection period; and
    - outputting (618) an instruction to a user to further move the detector (110) on the basis of the at least one determined quality value, the instruction referring to at last a part of the remaining detection period (622), **characterized in that** the detector data contain information about the position and/or orientation of the detector.

2. The method of image generation according to claim 1,
wherein the detector (110) is freely movable and typically a freehand detector.

3. The method of image generation according to any one of the preceding claims, the method further comprising:

    - continuously collecting detector data for image generation by the evaluating system (300) of the image generating apparatus during the detection period (622).

4. The method of image generation according to any one of the preceding claims, the method further comprising:

    - outputting the at least one determined quality value to a user.

**5.** The method of image generation according to any one of the preceding claims, the method further comprising:

- outputting an alert to a user, if the at least one quality value fails to reach a threshold value.

**6.** The method of image generation according to any one of the preceding claims, wherein outputting an instruction to a user to further move the radiation detector comprises the outputting of such a position and/or orientation of the detector (110) which, if adopted by the detector, would most strongly improve image generation according to at least one quality value pursuant to a prognosis.

**7.** The method of image generation according to any one of the preceding claims, wherein outputting the instruction to the user and/or outputting the at least one determined quality value is performed on an output system with at least one outputting unit in a visual, acoustical or haptic manner or by combinations thereof.

**8.** The method of image generation according to any one of the preceding claims, the method further comprising:

- collecting body data by means of the evaluating system (300) with respect to respiration and/or heartbeat of a living being, e.g. comprising respiration frequency and/or heartbeat frequency, and/or body data comprising form, position and/or orientation of the body, wherein the body data with respect to respiration and/or heartbeat are optionally synchronized with the body data with respect to form, position and/or orientation of the body; and
- modifying an image generation rule on the basis of the collected body data.

**9.** The method of image generation according to any one of the preceding claims, the method further comprising:

- acquiring data of medical instruments (120) by means of the detecting system, and typically generating an instrument image on the basis of the collected instrument data by means of the evaluating system; and
- guiding the user, while employing the medical instruments, by means of a guiding system on the basis of the instrument data.

**10.** An image generating apparatus for image generation, comprising:

- a movable detector (110) for detecting radioactive radiation during a detection period (622), and
- an evaluating system (300), comprising:

an interface system (306) for transmitting to the evaluating system detector data including information on the detected radioactive radiation and the detector's position and/or orientation for image generation, a data memory section (320) for storing the detector data, and a program memory section (330) including a program for repeatedly determining at least one quality value with respect to image generation from the detector data during the detection period; and the image generating apparatus further comprising:

- an output system (400) comprising at least one outputting unit (410), wherein the at least one outputting unit (410) comprises or is for outputting an instruction to a user to further move the detector on the basis of the at least one determined quality value, wherein the instruction relates to at least a part of the remaining detection period (622).

**11.** The image generating apparatus for image generation according to claim 10, wherein the detector (110) is freely movable and typically a freehand detector.

**12.** The image generating apparatus for image generation according to any one of claims 10 - 11, wherein the interface system (306) is an interface system for continuously transmitting to the evaluating system (300) detector data including information on the detected radiation and including information on the detector's (110) position and/or orientation for image generation.

**13.** The image generating apparatus for image generation according to any one of claims 10 - 12, wherein the at least one outputting unit (410) comprises an outputting unit for outputting the at least one determined quality value to a user and/or an alert to a user, if the at least one quality value fails to meet at least one quality criterion.

**14.** The image generating apparatus for image generation according to any one of claims 10 - 13, wherein the at least one outputting unit (410) comprises an outputting unit for outputting such a position and/or orientation of the detector which, if adopted by the detector, would most strongly improve image generation according to at least one quality value pursuant to a prognosis.

**15.** The image generating apparatus (1) for image generation according to any one of claims 10 - 14, the image generating apparatus further comprising:

- an acquisition system (200) for acquiring detector data on the detector's position and/or orientation,

wherein the acquisition system (200) further comprises:

- at least one sensor for acquiring body data with respect to respiration and/or heartbeat of a living being, e.g. including respiration frequency and/or heartbeat frequency, and/or

- an acquisition unit for acquiring body data of the living being, the data comprising form, position and/or orientation of the body,

and wherein the evaluating system further comprises:

- an optional program memory section including a program for collecting body data of the living being in a synchronized manner, and

- a program memory section including a program for modifying an image generation rule on the basis of the collected body data.

**Revendications**

**1.** Procédé de formation d'image par un appareil de formation d'image (1), comprenant :

- la détection (614) de rayonnement radioactif par un détecteur mobile (110) pendant une période de détection (622) ;

- la collecte (615) de données de détecteur pour la formation d'image par un système d'évaluation (300) de l'appareil de formation d'image (1), les données de détecteur comprenant des informations relatives au rayonnement détecté ;

- la détermination répétée (616) d'au moins une valeur de qualité à partir des données de détecteur collectées, par le système d'évaluation pendant la période de détection ; et

- la sortie (618) d'une instruction à un usager en vue de la poursuite du déplacement du détecteur (110) en fonction de l'au moins une valeur de qualité déterminée, l'instruction se rapportant au moins à une partie de la période de détection (622) restante, **caractérisé en ce que** les données de détecteur comprennent des informations relatives à la position et/ou l'orientation du détecteur.

**2.** Procédé de formation d'image selon la revendication 1, dans lequel le détecteur (110) est librement mobile, et est typiquement un détecteur main libre.

**3.** Procédé de formation d'image selon l'une des revendications précédentes, dans lequel le procédé comprend en outre :

- la collecte continue de données de détecteur pour la formation d'image par le système d'évaluation (300) de l'appareil de formation d'image pendant la période de détection (622).

**4.** Procédé de formation d'image selon l'une des revendications précédentes, le procédé comprenant en outre :

- la sortie de l'au moins une valeur de qualité déterminée à un usager.

**5.** Procédé de formation d'image selon l'une des revendications précédentes, le procédé comprenant en outre :

- la sortie d'un avertissement à un usager si l'au moins une valeur de qualité n'atteint pas une valeur seuil.

**6.** Procédé de formation d'image selon l'une des revendications précédentes, dans lequel la sortie d'une instruction à un usager en vue de la poursuite du déplacement du détecteur de rayonnement comprend la sortie de la position et/ou de l'orientation du détecteur (110) dont l'adoption par le détecteur améliorerait le plus fortement selon une prévision la formation d'image selon au moins une valeur de qualité.

**7.** Procédé de formation d'image selon l'une des revendications précédentes, dans lequel la sortie de l'instruction à l'usager et/ou la sortie de l'au moins une valeur de qualité déterminée sur un système de sortie avec au moins une unité de sortie s'effectue visuellement, acoustiquement, haptiquement ou par des combinaisons de ces modes.

**8.** Procédé de formation d'image selon l'une des revendications précédentes, le procédé comprenant en outre :

- la collecte, par le système d'évaluation (300), de données corporelles relatives à la respiration et/ou au rythme cardiaque d'un être vivant, lesquelles comprennent en particulier la fréquence respiratoire et/ou la fréquence cardiaque, et/ou de données corporelles comprenant la forme, la position et/ou l'orientation du corps, les données corporelles relatives à la respiration et/ou au rythme cardiaque étant facultativement synchronisées avec les données corporelles relatives à la forme, la position et/ou l'orientation du corps ; et

- la modification d'une consigne de formation d'image sur la base des données corporelles collectées.

9. Procédé de formation d'image selon l'une des revendications précédentes, le procédé comprenant en outre :

- l'acquisition de données d'instruments médicaux (120) par le système d'acquisition, et typiquement la formation, par le système d'évaluation, d'une image d'instruments sur la base des données d'instruments collectées ; et
- le guidage de l'usager lors de l'utilisation des instruments médicaux par un système de guidage sur la base des données d'instruments.

10. Appareil de formation d'image pour la formation d'image, lequel comprend :

- un détecteur mobile (110) pour la détection de rayonnement radioactif pendant une période de détection (622), et
- un système d'évaluation (300), lequel comprend :

un système d'interface (306) pour la transmission au système d'évaluation de données de détecteur avec des informations relatives au rayonnement radioactif détecté et relatives à la position et/ou l'orientation du détecteur pour la formation d'image, une section d'enregistrement de données (320) pour l'enregistrement des données de détecteur, et une section d'enregistrement de programme (330) comprenant un programme pour la détermination répétée d'au moins une valeur de qualité eu égard à la formation d'image à partir des données de détecteur pendant la période de détection ; et l'appareil de formation d'image comprenant en outre :

- un système de sortie (400), lequel comprend au moins une unité de sortie (410), l'au moins une unité de sortie (410) comprenant une unité de sortie destinée à sortir une instruction à un usager en vue de la poursuite du déplacement du détecteur en fonction de l'au moins une valeur de qualité déterminée, l'instruction se rapportant au moins à une partie de la période de détection (622) restante.

11. Appareil de formation d'image pour la formation d'image selon la revendication 10, dans lequel le détecteur (110) est librement mobile, et est typiquement un détecteur main libre.

12. Appareil de formation d'image pour la formation d'image selon l'une des revendications 10 - 11, dans lequel le système d'interface (306) est un système d'interface pour la transmission continue de données de détecteur au système d'évaluation (300) avec des informations relatives au rayonnement détecté et avec des informations relatives à la position et/ou l'orientation du détecteur (110) pour la formation d'image.

13. Appareil de formation d'image pour la formation d'image selon l'une des revendications 10 à 12, dans lequel l'au moins une unité de sortie (410) comprend une unité de sortie destinée à sortir l'au moins une valeur de qualité déterminée à un usager et/ou un avertissement à un usager si l'au moins une valeur de qualité ne remplit pas au moins un critère de qualité.

14. Appareil de formation d'image pour la formation d'image selon l'une des revendications 10 à 13, dans lequel l'au moins une unité de sortie (410) comprend une unité de sortie destinée à sortir la position et/ou l'orientation du détecteur dont l'adoption par le détecteur améliorerait le plus fortement selon une prévision la formation d'image selon au moins une valeur de qualité.

15. Appareil de formation d'image (1) pour la formation d'image selon l'une des revendications 10 à 14, dans lequel l'appareil de formation d'image comprend en outre :

- un système d'acquisition (200) destiné à acquérir des données de détecteur relatives à la position et/ou l'orientation du détecteur, le système d'acquisition (200) comprenant en outre :
- au moins un capteur destiné à acquérir des données corporelles relatives à la respiration et/ou au rythme cardiaque d'un être vivant, lesquelles comprennent en particulier la fréquence respiratoire et/ou la fréquence cardiaque, et/ou
- une unité d'acquisition destiné à acquérir des données corporelles de l'être vivant, les données comprenant la forme, la position et/ou l'orientation du corps, et le système d'évaluation comprenant en outre :
- facultativement une section d'enregistrement de programme comprenant un programme pour la collecte synchronisée de données corporelles de l'être vivant et
- une section d'enregistrement de programme comprenant un programme pour la modification d'une consigne de formation d'image sur la base des données corporelles collectées.

**100** Detektorsystem

Detektor

**110**

**200** Erfassungssystem

Erfassungseinheit

**210**

**300** Auswertesystem

Speichereinheit **310**

Recheneinheit

**350** Bilderzeugungsapparat 1

**400** Ausgabesystem

Ausgabeeinheit

**410**

**500** Führungssystem

Führungseinheit

**510**

**Abbildung 1**

Detektorsystem 100

110

120

10

Abbildung 2

EP 2 165 215 B1

Abbildung 3

EP 2 165 215 B1

Auswertesystem 300

| Speichersystem | 302 |
| Speichereinheit | 310 |
| Datenspeicherabschnitt | 320 |
| Programmspeicherabschnitt | 330 |

| Rechensystem | 304 |
| Recheneinheit | 350 |

| Schnittstellensystem | 306 |
| Detektorsystemschnittstelle | 306A |
| Detektorschnittstelle | 380 |
| Erfassungssystemschnittstelle | 306B |
| Erfassungseinheitsschnittstelle | 390 |

4

**Abbildung 4**

| Programmspeicherabschnitt | 330 |

| Programm zum Ermitteln mindestens eines Qualitätswerts aus den Detektordaten | 330A |

| Programmspeicherabschnitt | 332 |

| Programm zum Ermitteln einer Bilderzeugungsvorschrift auf der Grundlage von Detektordaten unter Berücksichtigung eines Detektionmodells | 332A |

| Programmspeicherabschnitt | 334 |

| Programm | 334A |

| Programmteil zum Ermitteln mindestens eines Qualitätswerts aus den Detektordaten | 334B |

| Programmteil zum wiederholten Ermitteln mindestens eines Qualitätswerts aus den Detektordaten | 334C |

**Abbildung 5**

EP 2 165 215 B1

Ausgabesystem 400

410

420

Abbildung 6

EP 2 165 215 B1

430

**Abbildung 7**

510

40

30

Führungssystem 500

Abbildung 8

EP 2 165 215 B1

**Abbildung 9**

**Abbildung 10**

**Abbildung 11**

629

620

Warnsignal

Zeit

Detektionszeitraum

622

624

Qualitätswertermittlungszeitraum

626

628

**Abbildung 12**

**Abbildung 13**

**Abbildung 14**

EP 2 165 215 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6510336 B
- EP 1237012 A
- WO 2007131561 A